(19) Europäisches Patentamt
European Patent Office
Office européen des brevets

(11) Veröffentlichungsnummer : **0 008 391**
**B1**

# (12) EUROPÄISCHE PATENTSCHRIFT

(45) Veröffentlichungstag der Patentschrift : **03.02.82**

(51) Int. Cl.: **C 07 D 403/04**, A 61 K 31/50 // C07D403/04, 237/04, 235/04)

(21) Anmeldenummer : **79102758.4**

(22) Anmeldetag : **01.08.79**

## (54) Benzimidazole, deren Herstellung und diese Verbindungen enthaltende Arzneimittel.

(30) Priorität : **25.08.78 DE 2837161**
**01.06.79 DE 2922336**

(43) Veröffentlichungstag der Anmeldung : **05.03.80 (Patentblatt 80/05)**

(45) Bekanntmachung des Hinweises auf die Patenterteilung : **03.02.82 Patentblatt 82/05**

(84) Benannte Vertragsstaaten : **AT BE CH DE FR GB IT LU NL SE**

(56) Entgegenhaltungen :
**DE - A - 2 427 943**
**GB - A - 1 466 547**

(73) Patentinhaber : **Dr. Karl Thomae GmbH**
**Postfach 720**
**D-7950 Biberach (Riss) (DE)**

(72) Erfinder : **Austel, Volkhard, Dr. Dipl.-Chem.**
**Kapellenweg 7**
**D-7950 Biberach 1 (DE)**
Erfinder : **Heider, Joachim, Dr. Dipl.-Chem.**
**Am Hang 3**
**D-7951 Warthausen 1 (DE)**
Erfinder : **Eberlein, Wolfgang, Dr. Dipl.-Chem.**
**Obere Au 6**
**D-7950 Biberach 1 (DE)**
Erfinder : **Diederen, Willi, Dr.**
**Haldenstrasse 1a**
**D-7950 Biberach 1 (DE)**
Erfinder : **Haarmann, Walter, Dr.**
**Schillerholzweg 8**
**D-7950 Biberach 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 391 B1

Benzimidazole, deren Herstellung und diese Verbindungen enthaltende Arzneimittel

In der GB-PS 1 466 547 werden bereits u.a. durch einen Pyridazinrest, welcher in 2- und/oder 4-Stellung jeweils durch eine Methylgruppe substituiert ist, substituierte Benzimidazole beschrieben, welche eine blutdrucksenkende, antithrombotische und cardiotonische Wirkung aufweisen.

Es wurde nun gefunden, daß die durch einen Pyridazinring, welcher in 5-Stellung durch einen Alkylrest substituiert ist, substituierten Benzimidazole eine größere Wirkungsstärke und/oder bessere orale Resorption aufweisen.

Gegenstand der vorliegenden Erfindung sind somit neue in 5- oder 6-Stellung durch einen Pyridazinring, der in 5-Stellung durch einen Alkylrest substituiert ist, substituierte Benzimidazole der allgemeinen Formel

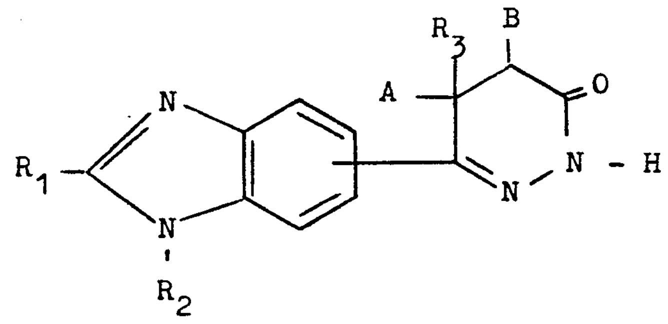

(I)

deren physiologisch verträgliche Säureadditionssalze mit anorganischen oder organischen Säuren, Verfahren zu ihrer Herstellung und diese Verbindungen enthaltende Arzneimittel.

Die Verbindungen der obigen allgemeinen Formel I und deren physiologisch verträgliche Säureadditionssalze weisen wertvolle pharmakologische Eigenschaften auf, neben einer antiviralen, Interferon-induzierenden und Ulcus-hemmenden Wirkung, insbesondere cardiovasculäre Wirkungen, nämlich cardiotonische, blutdrucksenkende und/oder antithrombotische.

In der obigen allgemeinen Formel I bedeutet

A und B je ein Wasserstoffatom oder zusammen eine weitere Bindung,

$R_1$ ein Wasserstoffatom, die Trifluormethylgruppe, eine Alkylgruppe mit 1 bis 11 Kohlenstoffatomen, eine Cycloalkylgruppe mit 3 bis 7 Kohlenstoffatomen, eine gegebenenfalls durch eine Alkylgruppe mit 1 bis 6 Kohlenstoffatomen substituierte Hydroxy- oder Mercaptogruppe, eine Phenylalkylgruppe mit 1 bis 3 Kohlenstoffatomen im Alkylteil oder eine Phenylgruppe, die durch ein Halogenatom, eine Alkylgruppe mit 1 bis 4 Kohlenstoffatomen, eine Alkylsulfinylgruppe mit 1 bis 6 Kohlenstoffatomen und/oder eine gegebenenfalls durch eine Alkylgruppe mit 1 bis 6 Kohlenstoffatomen substituierte Hydroxy- oder Mercaptogruppe mono-, di- oder trisubstituiert sein kann, wobei die Substituenten des Phenylkerns gleich oder verschieden sein können,

$R_2$ ein Wasserstoffatom, eine Alkylgruppe mit 1 bis 6 Kohlenstoffatomen, eine Cycloalkylgruppe mit 3 bis 6 Kohlenstoffatomen oder eine Phenylalkylgruppe mit 1 bis 3 Kohlenstoffatomen im Alkylteil und

$R_3$ eine Alkylgruppe mit 1 bis 6 Kohlenstoffatomen.

Für die bei der Definition der Reste $R_1$, $R_2$ und $R_3$ eingangs erwähnten Bedeutungen kommt somit für $R_1$ insbesondere die Bedeutung des Wasserstoffatoms, der Trifluormethyl-, Methyl-, Äthyl-, Propyl-, Isopropyl-, n-Butyl-, Isobutyl-, sec.Butyl-, tert.Butyl-, n-Pentyl-, 2-Pentyl-, tert.Pentyl-, Isopentyl-, n-Hexyl-, n-Undecyl-, Cyclopropyl-, Cyclobutyl-, Cyclopentyl-, Cyclohexyl-, Cycloheptyl-, Hydroxy-, Methoxy-, Äthoxy-, Propoxy-, Isopropoxy-, Mercapto-, Methylmercapto-, Äthylmercapto-, Propylmercapto-, Isopropylmercapto-, Hexylmercapto-, Benzyl-, 1-Phenyläthyl-, 2-Phenyläthyl-, 1-Phenylpropyl-, 2-Phenylpropyl-1, 1-Phenyl-2-propyl-, 3-Phenylpropyl-, Phenyl-, Fluorphenyl-, Difluorphenyl-, Chlorphenyl-, Dichlorphenyl-, Bromphenyl-, Dibromphenyl-, Methylphenyl-, Dimethylphenyl-, tert.Butylphenyl-, Hydroxyphenyl-, Dihydroxyphenyl-, Methoxyphenyl-, Dimethoxyphenyl-, Trimethoxyphenyl-, n-Hexoxyphenyl-, Mercaptophenyl-, Dimercaptophenyl-, Methylmercaptophenyl-, Dimethylmercaptophenyl-, Methylsulfinylphenyl-, Dimethylsulfinylphenyl-, Fluor-hydroxyphenyl-, Fluormethoxyphenyl-, Fluormercaptophenyl-, Fluor-methylmercaptophenyl-, Chlor-hydroxyphenyl-, Chlor-methoxyphenyl-, Chlormercaptophenyl-, Chlor-methylmercaptophenyl-, Brom-hydroxyphenyl-, Brom-methoxyphenyl-, Brommercaptophenyl-, Brom-methylmercaptophenyl-, Hydroxy-methoxyphenyl-, Hydroxy-mercaptophenyl-, Hydroxy-methylmercaptophenyl-, Methoxy-mercaptophenyl-, Methoxy-methylmercaptophenyl- oder Methoxy-methylsulfinylphenylgruppe,

für $R_2$ die des Wasserstoffatoms, der Methyl-, Äthyl-, Propyl-, Isopropyl-, Butyl-, Pentyl-, Isopentyl-, Cyclopropyl-, Cyclobutyl-, Cyclopentyl-, Cyclohexyl-, Benzyl-, 1-Phenyläthyl-, 2-Phenyläthyl- oder 3-Phenylpropylgruppe und

für $R_3$ die der Methyl-, Äthyl-, n-Propyl-, Isopropyl-, n-Butyl-, Isobutyl-, sec.Butyl- oder tert.Butylgruppe in Betracht.

Bevorzugte Verbindungen der allgemeinen Formel I sind diejenigen, in der

A und B je ein Wasserstoffatom oder zusammen eine weitere Bindung,

$R_1$ ein Wasserstoffatom, die Methyl-, Isopropyl-, n-Pentyl-, 2-Pentyl-, n-Hexyl-, n-Undecyl-, Cyclopropyl-, Cyclohexyl-, Cycloheptyl-, Hydroxy-, Äthoxy-, Mercapto-, Methylmercapto-, Isopropylmercapto-, Hexylmercapto-, Trifluormethyl-, 1-Phenyläthyl-, 1-Phenyl-2-propyl-, Phenyl-, 2-Fluorphenyl-, 4-Chlorphenyl-, 4-Hydroxyphenyl-, 2-Methoxyphenyl-, 3-Methoxyphenyl-, 4-Methoxyphenyl-, 2,4-Dimethoxyphenyl-, 3,4-Dimethoxyphenyl-, 4-n-Hexyloxyphenyl-, 3,4,5-Trimethoxyphenyl-, 2-Methoxy-4-methylmercaptophenyl-, 4-Methylphenyl-, 4-tert.Butylphenyl-, 2-Methoxy-4-methylsulfinylphenyl- oder 2-Brom-5-methoxyphenylgruppe,

$R_2$ ein Wasserstoffatom, die Methyl-, 3-Methylbutyl-, Cyclopropyl-, Cyclohexyl- oder Benzylgruppe und

$R_3$ die Methyl-, Äthyl- oder n-Butylgruppe bedeuten, sowie deren physiologisch verträgliche Säureadditionssalze mit anorganischen oder organischen Säuren.

Besonders bevorzugte Verbindungen der allgemeinen Formel I sind jedoch diejenigen, in der

A und B je ein Wasserstoffatom oder zusammen eine weitere Bindung,

$R_1$ ein Wasserstoffatom, die Methyl-, 2-Pentyl-, 1-Phenyläthyl-, Äthoxy-, Methylmercapto-, 4-Methylphenyl-, 2-Fluorphenyl-, 4-Methoxyphenyl-, 4-Hexyloxyphenyl- oder 2,4-Dimethoxyphenylgruppe,

$R_2$ ein Wasserstoffatom, die Methyl- oder Benzylgruppe und

$R_3$ die Methylgruppe bedeuten, sowie deren physiologisch verträgliche Säureadditionssalze mit anorganischen oder organischen Säuren.

Desweiteren können die neuen Benzimidazole der vorliegenden Erfindung aufgrund ihres optisch aktiven Kohlenstoffatoms in 5-Stellung des Pyridazinringes, wenn A und B je ein Wasserstoffatom darstellen, in Form ihres Racemates oder ihrer optisch aktiven Antipoden auftreten.

Erfindungsgemäß lassen sich die neuen Benzimidazole, deren Racemate und optisch aktive Antipoden, wenn A und B je ein Wasserstoffatom darstellen, nach folgenden Verfahren herstellen :

a) Cyclisierung einer gegebenenfalls im Reaktionsgemisch hergestellten Verbindung der allgemeinen Formel

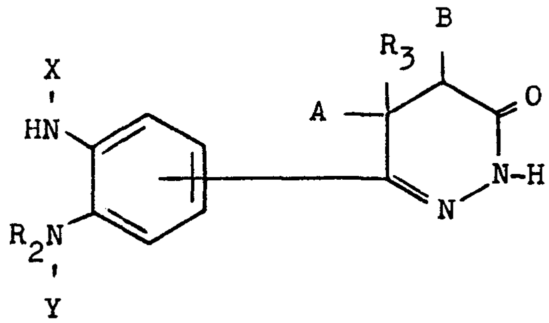

(II)

in der

A, B, $R_2$ und $R_3$ wie eingangs definiert sind, einer der Reste X oder Y ein Wasserstoffatom und der andere der Reste X oder Y oder beide der Reste X und Y je eine Gruppe der Formel

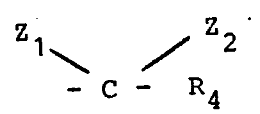

darstellen, in der

$Z_1$ und $Z_2$, die gleich oder verschieden sein können, gegebenefalls substituierte Aminogruppe oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen und

$R_4$ die für $R_1$ eingangs erwähnten Bedeutungen besitzt oder eine gegebenenfalls durch eine Alkylgruppe mit 1 bis 3 Kohlenstoffatomen substituierte Aminogruppe darstellen, oder deren Alkalisalze, wenn $R_4$ die Mercaptogruppe darstellt.

Die Cyclisierung wird zweckmäßigerweise in einem Lösungsmittel wie Äthanol, Isopropanol, Eisessig, Chlorbenzol, Toluol, Xylol, Glycol, Glycolmonomethyläther, Diäthylenglycoldimethyläther, Dimethylformamid, Tetralin oder in einem Überschuß des zur Herstellung der Verbindung der allgemeinen Formel II verwendeten Acylierungsmittels, z.B. in $R_1CN$, $(R_1CO)_2O$, $R_1COOH$, $R_1CSOH$ oder $R_1CSSH$ und deren Estern, Orthoestern, Amiden, Halogeniden oder Methojodiden, bei erhöhten Temperaturen, z.B. bei Temperaturen zwischen 0 und 250 °C, gegebenenfalls in Gegenwart eines Kondensationsmittels wie Phosphoroxychlorid, Thionylchlorid, Sulfurylchlorid, Schwefelsäure, Salzsäure, Phosphorsäure,

Polyphosphorsäure, p-Toluolsulfonsäure, Eisessig, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder ohne Kondensationsmittel durchgeführt werden.

Besonders vorteilhaft wird die Umsetzung jedoch in der Weise durchgeführt, daß ein entsprechendes 6-(Acylamino-nitro-phenyl)-pyridazin-3-on durch Reduktion, beispielsweise durch Reduktion mit Wasserstoff in Gegenwart eines Hydrierungskatalysators wie Raney-Nickel, Platin oder Palladium/Kohle, durch Reduktion mit Metallen wie Eisen, Zinn oder Zink oder durch Reduktion mit Metallsalzen wie Eisen-(II)-sulfat, Zinn-(II) chlorid oder Chrom-(II)-chlorid oder durch Reduktion mit Hydrazin in Gegenwart von Raney-Nickel, in eine entsprechende Verbindung der allgemeinen Formel II übergeführt wird, welche gegebenenfalls im gleichen Reaktionsgemisch erforderlichenfalls in Gegenwart einer Säure wie Salzsäure, Schwefelsäure, Phosphorsäure, Essigsäure oder einer Carbonsäure der Formel $R_1COOH$, oder in Gegenwart eines Kondensationsmittels wie Phosphoroxychlorid oder in Gegenwart einer Base wie Kalium-äthylat gegebenenfalls in einem Lösungsmittel wie Äthanol, Isopropanol, Glycol, Dimethylformamid, Dimethylsulfoxid oder Chlorbenzol bei Temperaturen zwischen 0 und 250 °C, cyclisiert wird.

Zur Herstellung einer Verbindung der allgemeinen Formel I, in der $R_1$ die Mercaptogruppe darstellt, wird die Umsetzung zweckmäßigerweise in der Weise durchgeführt, daß eine Verbindung der allgemeinen Formel II in situ durch Umsetzung einer entsprechenden 7,8-Diaminoverbindung mit Schwefelkohlenstoff in Gegenwart eines Alkalialkoholats, z.B. in Gegenwart von Kalium-äthylat, in einem alkoholischen Lösungsmittel wie Äthanol hergestellt wird. Die anschließende Cyclisierung erfolgt durch einfaches Erhitzen des Reaktionsgemisches, vorzugsweise durch Erhitzen auf die Siedetemperatur des Reaktionsgemisches.

b) Umsetzung einer Carbonsäure der allgemeinen Formel

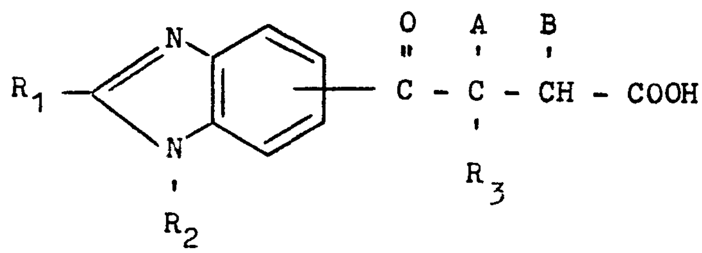

(III)

in der

A, B, $R_1$, $R_2$ und $R_3$ wie eingangs definiert sind, oder deren Ester, Amide oder Halogenide mit Hydrazin.

Die Umsetzung wird zweckmäßigerweise in einem Lösungsmittel wie Äthanol, Isopropanol, Eisessig, Propionsäure und/oder in einem Überschuß von Hydrazin bzw. Hydrazin-hydrat bei erhöhten Temperaturen, z.B. bei Temperaturen zwischen 0 und 150 °C, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durchgeführt werden.

Eine gemäß den Verfahren a) oder b) erhaltene Verbindung der allgemeinen Formel I, in der A und B je ein Wasserstoffatom darstellen, kann anschließend gewünschtenfalls durch Dehydrierung in eine Verbindung der allgemeinen Formel I, in der A und B zusammen eine weitere Bindung darstellen, übergeführt werden und/oder eine erhaltene Verbindung der allgemeinen Formel I, in der $R_1$ die Hydroxy- oder Mercaptogruppe oder eine Phenylgruppe, die durch eine Hydroxy- und/oder Mercaptogruppe mono, di- oder trisubstituiert ist, und/oder $R_2$ ein Wasserstoffatom darstellt, mittels Alkylierung in eine entsprechende Alkoxy-, Alkylmercapto- und/oder Alkylverbindung der allgemeinen Formel I übergeführt werden, und/oder eine erhaltene Verbindung der allgemeinen Formel I, in der $R_1$ eine durch mindestens eine Alkylmercaptogruppe substituierte Phenylgruppe darstellt, mittels Oxidation in eine entsprechende Alkylsulfinylphenylverbindung der allgemeinen Formel I übergeführt werden, und/oder eine erhaltene Verbindung der allgemeinen Formel I, in der $R_1$ eine Alkylmercaptogruppe darstellt, mittels Oxidation und anschließende hydrolytische Abspaltung der gebildeten Alkylsulfinyl- bzw. Alkylsulfonylgruppe in eine entsprechende Hydroxyverbindung der allgemeinen Formel I übergeführt werden.

Die nachträgliche Dehydrierung wird mit einem Dehydrierungsmittel wie Brom, Phosphorpentachlorid, 3-Nitro-benzolsulfonsaurem Natrium, Chromtrioxid, N-Bromsuccinimid, Wasserstoffperoxid oder Natriumnitrit in einem Lösungsmittel wie Eisessig, Propionsäure, Wasser/Eisessig oder Nitrobenzol bei Temperaturen zwischen 0 und 100 °C, vorzugsweise jedoch bei Temperaturen zwischen 50 und 80 °C, durchgeführt.

Die nachträgliche Alkylierung wird mit einem Alkylierungsmittel wie einem Alkylhalogenid, Dialkylsulfat, Trialkyloxonium-tetrafluorborat oder Diazoalkan, z.B. mit Methyljodid, Dimethylsulfat, Diäthylsulfat, Äthylbromid oder Diazomethan, zweckmäßigerweise in einem Lösungsmittel wie Methanol, Äthanol, Methanol/Wasser, Diäthyläther oder Dioxan gegebenenfalls in Gegenwart einer Base wie Natriumbicarbonat, Natriumcarbonat, Natriumhydroxid, Natriummethylat oder Kaliumcarbonat bei Temperaturen bis zur Siedetemperatur des verwendeten Lösungsmittels durchgeführt.

Die anschließende Oxidation wird vorzugsweise in einem Lösungsmittel wie Eisessig oder Trifluoressigsäure und zweckmäßigerweise mit einem Äquivalent des verwendeten Oxidationsmittels wie Wasserstoffperoxid, falls eine Alkylsulfinylphenylverbindung hergestellt wird, bei Temperaturen zwischen 0 und 50 °C, vorzugsweise jedoch bei Raumtemperatur, durchgeführt.

Ferner können die erhaltenen Verbindungen der allgemeinen Formel I, in der A und B je ein Wasserstoffatom darstellen, in ihre optisch aktiven Antipoden mittels Racematspaltung aufgetrennt werden. Die Racematspaltung wird zweckmäßigerweise durch fraktionierte Kristallisation der entsprechenden Salze mit optisch aktiven Säuren, wie Weinsäure, Dibenzoylweinsäure, Äpfelsäure, Camphersäure oder Camphersulfonsäure durchgeführt.

Desweiteren können die erhaltenen Verbindungen der allgemeinen Formel I gewünschtenfalls in ihre physiologisch verträglichen Salze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III erhält man nach literaturbekannten Verfahren (siehe GB-PS 1 466 547). So erhält man beispielsweise eine Verbindung der allgemeinen Formel III durch Umsetzung einer Verbindung der allgemeinen Formel

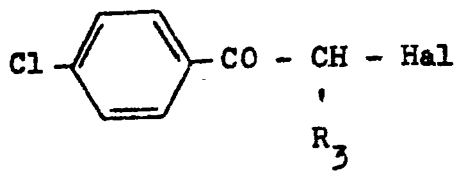

(IV)

mit Malonester. Die so erhaltene Verbindung wird anschließend verseift, decarboxyliert, nitriert, das Chloratom durch eine entsprechende Aminogruppe ersetzt, acyliert, die Nitrogruppe reduziert und zu dem gewünschten Benzimidazol cyclisiert.

Die Verbindungen der vorliegenden Anmeldung unterscheiden sich von denjenigen der Britischen Patentschrift 1.466.547 durch den Alkylsubstituenten $R_3$ in 5-Stellung des Pyridazinringes, außerdem weisen die neuen Benzimidazole überraschenderweise eine größere Wirkungsstärke und/oder bessere orale Resorption auf. Die neuen Verbindungen der vorliegenden Anmeldung weisen somit, wie bereits eingangs erwähnt ist, bei einer guten oralen Resorption überlegene pharmakologische Eigenschaften auf, neben einer antiviralen, Interferon-induzierenden und Ulcus-hemmenden Wirkung, insbesondere cardiovasculäre Wirkungen, nämlich cardiotonische, blutdrucksenkende und/oder antithrombotische.

Beispielsweise wurden die Verbindungen

A = 2-Methyl-5(6)-(5-methyl-3-oxo-4,5-dihydro-2H-6-pyridazinyl)-benzimidazol,
B = 2-Methyl-5(6)-(5-methyl-3-oxo-2H-6-pyridazinyl)-benzimidazol,
C = 2-Trifluormethyl-5(6)-(5-methyl-3-oxo-4,5-dihydro-2H-6-pyridazinyl)-benzimidazol,
D = 5(6)-(5-Methyl-3-oxo-4,5-dihydro-2H-6-pyridazinyl)-benzimidazol,
E = 2-(2-Fluorphenyl)-5(6)-(5-methyl-3-oxo-4,5-dihydro-2H-6-pyridazinyl)-benzimidazol,
F = 2-(2,4-Dimethoxy-phenyl)-5(6)-(5-methyl-3-oxo-4,5-dihydro-2H-6-pyridazinyl)-benzimidazol,
G = 2-Methylmercapto-5(6)-(5-methyl-3-oxo-4,5-dihydro-2H-6-pyridazinyl)-benzimidazol,
H = 2-(4-Methoxy-phenyl)-5(6)-(5-methyl-3-oxo-4,5-dihydro-2H-6-pyridazinyl)-benzimidazol,
I = 2-(4-Methyl-phenyl)-5(6)-(5-methyl-3-oxo-4,5-dihydro-2H-6-pyridazinyl)-benzimidazol,
K = 2-(2-Pentyl)-5(6)-(5-methyl-3-oxo-4,5-dihydro-2H-6-pyridazinyl)-benzimidazol-hydrochlorid,
L = 1-Methyl-2-(4-methoxy-phenyl)-5-(5-methyl-3-oxo-4,5-dihydro-2H-6-pyridazinyl)-benzimidazol
und
M = 1-Benzyl-2-methyl-5-(5-methyl-3-oxo-4,5-dihydro-2H-6-pyridazinyl)-benzimidazol im Vergleich zu
N = 2-Methyl-5(6)-(4-methyl-3-oxo-4,5-dihydro-2H-6-pyridazinyl)-benzimidazol,
O = 2-(2,4-Dimethoxy-phenyl-5(6)-(4-methyl-3-oxo-4,5-dihydro-2H-6-pyridazinyl)-benzimidazol,
P = 2-(4-Methoxy-phenyl)-5(6)-(4-methyl-3-oxo-4,5-dihydro-2H-6-pyridazinyl)-benzimidazol
und
Q = 2-(4-Methoxy-phenyl)-5(6)-(2,4-dimethyl-3-oxo-4,5-dihydro-2H-6-pyridazinyl)-benzimidazol (siehe GB-PS 1 466 547)

auf ihre biologischen Eigenschaften wie folgt untersucht :

1. Bestimmung der Thrombozytenaggregation nach Born und Cross (J. Physiol. *170,* 397 (1964)) :

Die Thrombozytenaggregation wurde in plättchenreichem Plasma gesunder Versuchspersonen gemessen. Hierbei wurde der Verlauf der Abnahme der optischen Dichte nach Zugabe von handelsüblichem Collagen der Firma Sigma, St. Louis/USA, welches 1 mg Collagen-Fibrillen pro ml enthält, photometrisch gemessen und registriert. Aus dem Neigungswinkel der Dichtekurve Wurde auf die Aggregationsgeschwindigkeit (Vmax) geschlossen. Der Punkt der Kurve, bei dem die größte Lichtdurchlässigkeit vorlag, diente zur Berechnung der « optical density » (O.D.). Zur maximalen

Aggregationsauslösung werden ca. 0,01 ml der Collagenlösung zu 1 ml plättchenreichem Plasma gegeben.

Die nachfolgende Tabelle enthält die gefundenen Werte :

Tabelle I

| Verbindung | $EC_{50}$ in μMol/l |
|---|---|
| A | 0,01 |
| B | 34 |
| C | 0,1 |
| D | 0,12 |

2. Bestimmung der blutdrucksenkenden und positiv inotropen Wirkung an der narkotisierten Katze :

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen.

Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 0,1, 0,5 oder 2,0 mg/kg i.v. Die Wirkungsdauer der untersuchten Substanzen beträgt jeweils mindestens eine Stunde.

Die nachfolgende Tabelle enthält die Mittelwerte :

Tabelle II

| Substanz | Dosis mg/kg i.v. | Blutdruckänderung in mmHg | Zunahme von dp/dt in % |
|---|---|---|---|
| A | 0,1 | − 26/24 | + 53 |
| B | 0,5 | + 18/15 | + 25 |
| C | 0,1 | − 65/35 | + 91 |
| D | 0,5 | − 55/48 | + 159 |
| E | 0,5 | − 8/10 | + 107 |
| F | 0,5 | − 28/33 | + 89 |
| G | 0,1 | − 36/40 | + 51 |
| H | 0,1 | + 3/0 | + 23 |
| I | 0,5 | + 33/15 | + 118 |
| K | 0,1 | − 10/13 | + 83 |
| L | 0,1 | + 14/9 | + 17 |
| M | 0,1 | − 37/33 | + 123 |
| N | 0,5 | + 5/3 | + 23 (*) |
| O | 0,5 | + 15/6 | + 10 (*) |
| P | 2,0 | − 35/35 | − 5 |
| Q | 2,0 | − 40/40 | − 26 |

(*) Wirkungsdauer : 5-20 Minuten.

3. Akute Toxizität :

Die akute Toxizität der zu untersuchenden Substanzen wurde an weißen Mäusen nach oraler Gabe einer einmaligen Dosis orientierend bestimmt (Beobachtungszeit : 14 Tage) :

Tabelle III

| Substanz | Akute Toxizität mg/kg p.o. |
|---|---|
| A | > 600 (1 von 3 Tieren gestorben) |
| C | > 450 (0 von 6 Tieren gestorben) |
| E | > 600 (0 von 6 Tieren gestorben) |
| H | ~ 600 (3 von 6 Tieren gestorben) |
| I | > 600 (0 von 6 Tieren gestorben) |

Aufgrund ihrer pharmakologischen Eigenschaften eignen sich die erfindungsgemäß hergestellten Verbindungen der allgemeinen Formel I und deren optisch aktive Antipoden sowie deren physiologisch verträgliche Säureadditionssalze mit anorganischen oder organischen Säuren zur Behandlung der chronischen Herzinsuffizienz oder der Angina Pectoris und/oder zur Prophylaxe arterieller Thromboembolien und arterieller Verschlußkrankheiten, sowie zur Behandlung von Ulcera und zur Bekämpfung von Viren und viraler Erkrankungen.

Hierzu lassen sich die neuen Benzimidazole gegebenenfalls in Kombination mit anderen Wirksubstanzen in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 × täglich 5-200 mg, vorzugsweise jedoch 15-150 mg.

Da die Verbindungen der allgemeinen Formel I in ihren tautomeren Formen 1H bzw. 3H vorliegen können, wurde der Substitionsort am Benzimidazolkern mit 5(6) bezeichnet.

Die nachfolgenden Beispiele sollen die Erfindung näher erläutern :

## Beispiel 1

2-(4-Methoxy-phenyl)-5(6)-(5-methyl-3-oxo-4,5-dihydro-2H-6-pyridazinyl)-benzimidazol-hydrochlorid

a) 3-[3-Nitro-4-(4-methoxy-benzoylamino)-benzoyl]-buttersäuremethylester

10 g 3-(3-Nitro-4-amino-benzoyl)-buttersäuremethylester werden mit 10,2 g 4-Anissäurechlorid in 100 ml Chlorbenzol bis zur vollständigen Umsetzung des 3-(3-Nitro-4-amino-benzoyl)-buttersäuremethylesters zum Rückfluß erhitzt. Das Reaktionsgemisch wird mit Aktivkohle verrührt, filtriert und das Filtrat mit Cyclohexan versetzt, wobei das Produkt auskristallisiert. Es wird abgesaugt und mit Cyclohexan und Petroläther gewaschen.

Ausbeute : 10,7 g (73 % der Theorie),
Schmelzpunkt : 107-117 °C.

b) 5-Methyl-6-[3-nitro-4-(4-methoxy-benzoylamino)-phenyl]-3-oxo-4,5-dihydro-2H-pyridazin

20 ml 80 %iges Hydrazinhydrat werden unter Rühren und Eiskühlung zu 150 ml Eisessig getropft. Nach dem Abkühlen werden 10,7 g 3-[3-Nitro-4-(4-methoxy-benzoylamino)-benzoyl]-buttersäuremethylester zugegeben und die Mischung 40 Minuten zum Rückfluß erhitzt. Nach dem Abkühlen wird mit 250 ml Eiswasser versetzt, der Niederschlag abgesaugt und mit Eiswasser gewaschen.

Ausbeute : 9,8 g (96 % der Theorie),
Schmelzpunkt : 219 °C.

c) 5-Methyl-6-[3-amino-4-(4-methoxy-benzoylamino)-phenyl]-3-oxo-4,5-dihydro-2H-pyridazin

9,8 g 5-Methyl-6-[3-nitro-4-(4-methoxy-benzoylamino)-phenyl]-3-oxo-4,5-dihydro-2H-pyridazin werden in 1 000 ml Äthanol gelöst und 23 Stunden bei Raumtemperatur mit Wasserstoff von 5 bar Druck in Gegenwart von 1 g 10 %iger Palladium-Kohle hydriert. Der entstandene Niederschlag wird abgesaugt und ohne Trennung vom Katalysator in der nächsten Stufe verwendet. Aus den Mutterlaugen kann eine weitere Fraktion des Produktes gewonnen werden.

Ausbeute : 8,5 g (94,4 % der Theorie).

d) 2-(4-Methoxy-phenyl)-5(6)-(5-methyl-3-oxo-4,5-dihydro-2H-6-pyridazinyl)-benzimidazol-hydrochlorid

Das unter c) erhaltene Produkt wird in 100 ml Eisessig 1,5 Stunden zum Rückfluß erhitzt und noch heiß durch eine Glasfritte filtriert. Das Filtrat wird mit 100 g Eis versetzt, der sich bildende orangegelbe Niederschlag wird abfiltriert und mit verdünnter Essigsäure gewaschen. Die vereinigten Filtrate werden mit Ammoniak alkalisch gestellt. Das ausgefallene Produkt wird, nachdem es kristallisiert ist, abgesaugt und über eine Kieselgelsäule (Elutionsmittel : Methylenchlorid/Äthanol = 50 : 1 und 25 : 1) gereinigt. Das Hydrochlorid wird aus Methanol mit ätherischer Salzäure gefällt.

Ausbeute : 4,45 g (49,8 % der Theorie),
Schmelzpunkt : 311 °C (Zersetzung).

## Beispiel 2

2-(4-Methoxy-phenyl)-5(6)-(5-methyl-3-oxo-4,5-dihydro-2H-6-pyridazinyl)-benzimidazol-hydrochlorid

a) 2-(4-Methoxy-phenyl)-5(6)-(1-oxo-2-methyl-3-methoxycarbonyl-1-propyl)-benzimidazol-hydrochlorid

9,2 g 5-methyl-6-[3-nitro-4-(4-methoxy-benzoylamino)-phenyl]-3-oxo-4,5-dihydro-2H-pyridazin werden in einem Gemisch aus 300 ml Methanol und 3 ml Eisessig 2,5 Stunden bei Raumtemperatur mit Wasserstoff von 5 bar Druck in Gegenwart von 2 g 10 %iger Palladium-Kohle hydriert. In das Reaktionsgemisch wird 2,5 Stunden Salzsäuregas unter Rückflußkochen eingeleitet. Danach wird mit Äther versetzt, abgesaugt und mit Äther gewaschen.

Ausbeute : 4,5 g (50,5 % der Theorie),
Schmelzpunkt : über 300 °C.

b) 2-(4-Methoxy-phenyl)-5(6)-(5-methyl-3-oxo-4,5-dihydro-2H-6-pyridazinyl)-benzimidazol-hydrochlorid

1 ml 80 %iges Hydrazinhydrat wird in 10 ml Eisessig gelöst und nach dem Abkühlen 0,43 g 2-(4-Methoxy-phenyl)-5(6)-(1-oxo-2-methyl-3-methoxycarbonyl-1-propyl)-benzimidazol-hydrochlorid zugegeben, das ausgefallene Produkt abgesaugt, mit Wasser gewaschen und über eine Kieselgelsäule (Elutionsmittel : Chloroform/Methanol = 50 : 1) gereinigt. Das Hydrochlorid wird aus Äthanol mit ätherischer Salzsäure gefällt.

Ausbeute : 0,3 g (70,3 % der Theorie),
Schmelzpunkt : 314 °C (Zersetzung).

## Beispiel 3

2-Methyl-5(6)-(5-methyl-3-oxo-4,5-dihydro-2H-6-pyridazinyl)-benzimidazol-hydrochlorid

29 g 5-Methyl-6-(3-nitro-4-acetamido-phenyl)-3-oxo-4,5-dihydro-2H-pyridazin werden in einem Gemisch aus 1 200 ml Methanol und 100 ml Eisessig bei Raumtemperatur 2,5 Stunden mit Wasserstoff von 5 bar Druck in Gegenwart von 5 g 10 %iger Palladium-Kohle hydriert. Der Katalysator wird abfiltriert und das Filtrat eingedampft. Der Rückstand wird in 400 ml Eisessig gelöst und 40 Minuten auf 100 °C erhitzt. Das Reaktionsgemisch wird auf 1 l Eis Gegossen und unter Kühlung durch Zusatz von konzentriertem Ammoniak auf pH 8 gebracht. Die ausgefallene freie Base überführt man schließlich durch Lösen in Methanol und Zugabe von ätherischer Salzsäure in das Hydrochlorid.

Ausbeute : 19,7 g (70,7 % der Theorie),
Schmelzpunkt : 308 °C.

## Beispiel 4

2-Methyl-5(6)-(5-methyl-3-oxo-2H-6-pyridazinyl)-benzimidazol

8 g 2-Methyl-5(6)-(5-methyl-3-oxo-4,5-dihydro-2H-6-pyridazinyl)-benzimidazol-hydrochlorid werden in 250 ml Eisessig suspendiert und unter Rühren bei 70 °C tropfenweise mit einer Lösung von 3 ml Brom in 30 ml Eisessig versetzt. Nach Beendigung der Zugabe wird noch 1 Stunde bei 70 °C gehalten, abgekühlt und das ausgefallene ölige Produkt durch Zugabe von 20 ml Wasser und kurzes Erwärmen kristallisiert. Das Kristallisat wird abgesaugt, mit konzentriertem Ammoniak verrieben, mit Wasser gewaschen und aus Methanol/Wasser umkristallisiert.

Ausbeute : 5,6 g (89 % der Theorie),
Schmelzpunkt : über 300 °C.

## Beispiel 5

2-(4-Methoxy-phenyl)-5(6)-(5-methyl-3-oxo-2H-6-pyridazinyl)-benzimidazol

Hergestellt analog Beispiel 4 aus 0,74 g 2-(4-Methoxy-phenyl)-5(6)-(5-methyl-3-oxo-4,5-dihydro-2H-6-pyridazinyl)-benzimidazol-hydrochlorid und 0,33 ml Brom. Das Produkt wird über eine Kieselgelsäule (Elutionsmittel : Chloroform/Methanol = 50 : 1) gereinigt.

Ausbeute : 0,35 g (52,6 % der Theorie),
Schmelzpunkt : über 300 °C.

## Beispiel 6

2-(2,4-Dimethoxy-phenyl)-5(6)-(5-methyl-3-oxo-4,5-dihydro-2H-6-pyridazinyl)-benzimidazol-hydrochlorid

a) 2-(2,4-Dimethoxy-phenyl)-5(6)-(1-oxo-2-methyl-3-äthoxycarbonyl-1-propyl)-benzimidazol-hydrochlorid

Hergestellt analog Beispiel 2a) aus 12,2 g 5-Methyl-6-[3-nitro-4-(2,4-dimethoxy-benzoylamino-phenyl]-3-oxo-4,5-dihydro-2H-pyridazin. Der Imidazolring wird mit äthanolischer Salzsäure geschlossen. Das Produkt wird nur grob gereinigt und in dieser Form weiter verarbeitet.
Ausbeute : 4 g (31,2 % der Theorie).

b) 2-(2,4-Dimethoxy-phenyl)-5(6)-(5-methyl-3-oxo-4,5-dihydro-2H-6-pyridazinyl)-benzimidazol-hydrochlorid

Hergestellt analog Beispiel 2b aus dem unter 6a gewonnenen Produkt.
Ausbeute : 0,78 g (21,1 % der Theorie),
Schmelzpunkt : 266 °C.

Beispiel 7

2-(4-Methyl-phenyl-5(6)-(5-methyl-3-oxo-4,5-dihydro-2H-6-pyridazinyl)-benzimidazol-hydrochlorid

Hergestellt analog Beispiel 2b aus 1,5 g 2-(4-Methyl-phenyl)-5(6)-(1-oxo-2-methyl-3-methoxycarbonyl-1-propyl)-benzimidazol-hydrochlorid.
Ausbeute : 0,24 g (16,5 % der Theorie),
Schmelzpunkt : 340 °C (Zersetzung).

Beispiel 8

2-(4-Methyl-phenyl-5(6)-(5-methyl-3-oxo-4,5-dihydro-2H-6-pyridazinyl)-benzimidazol-hydrochlorid

Hergestellt analog Beispiel 1d aus 21,5 g 5-Methyl-6-[2-amino-4-(4-methyl-benzoylamino)-phenyl]-3-oxo-4,5-dihydro-2H-pyridazin (Eine chromatographische Reinigung ist nicht erforderlich).
Ausbeute : 18,8 g (82,7 % der Theorie),
Schmelzpunkt : 340 °C (Zersetzung)

Beispiel 9

2-Cyclopropyl-5(6)-(5-methyl-3-oxo-4,5-dihydro-2H-6-pyridazinyl)-benzimidazol-hydrochlorid

Hergestellt analog Beispiel 6 aus 2,3 g 5-Methyl-6-(3-nitro-4-cyclopropylcarbonylamino-phenyl)-3-oxo-4,5-dihydro-2H-pyridazin.
Ausbeute : 1,35 g (61,2 % der Theorie),
Schmelzpunkt : 235-237 °C (Zersetzung).

Beispiel 10

1,2-Dimethyl-5(6)-(5-methyl-3-oxo-4,5-dihydro-2H-6-pyridazinyl)-benzimidazol

a) 5-Methyl-6-(3-amino-4-acetyl-methylamino-phenyl)-3-oxo-4,5-dihydro-2H-pyridazin

0,65 g 5-Methyl-6-(3-nitro-4-acetyl-methylamino-phenyl)-3-oxo-4,5-dihydro-2H-pyridazin werden in 30 ml Äthanol gelöst und mit 0,5 ml 80 %igem Hydrazinhydrat und 0,5 g Raney-Nickel versetzt und 10 Minuten auf 30 °C erwärmt. Der Katalysator wird abfiltriert, das Filtrat mit Eisessig versetzt und im Vakuum eingedampft. Der Rückstand wird direkt weiter verwendet.

b) 1,2-Dimethyl-5(6)-(5-methyl-3-oxo-4,5-dihydro-2H-6-pyridazinyl)-benzimidazol

Hergestellt analog Beispiel 1d aus dem unter 10a gewonnenen Produkt.
Ausbeute : 0,15 g (27,4 % der Theorie),
Schmelzpunkt : über 250 °C.

Beispiel 11

2-(2-Fluor-phenyl)-5(6)-(5-methyl-3-oxo-4,5-dihydro-2H-6-pyridazinyl)-benzimidazol-hydrochlorid

3,2 g 5-Methyl-6-[3-amino-4-(2-fluor-benzoylamino)-phenyl]-3-oxo-4,5-dihydro-2H-pyridazin werden in 150 ml Isopropanol suspendiert und in die Mischung unter Rückflußkochen 2 Stunden Salzsäuregas eingeleitet. Danach wird eingeengt und der Rückstand durch Verreiben mit Äther kristallisiert.
Ausbeute : 1,35 g (40 % der Theorie),
Schmelzpunkt : 295 °C.

Beispiel 12

2-Phenyl-5(6)-(5-methyl-3-oxo-4,5-dihydro-2H-6-pyridazinyl)-benzimidazol-hydrochlorid

Hergestellt analog Beispiel 11 aus 2,0 g 5-Methyl-6-(3-amino-4-benzoylamino-phenyl)-3-oxo-4,5-dihydro-2H-pyridazin.
Ausbeute : 0,85 g (40,5 % der Theorie),
Schmelzpunkt : 335 °C.

Beispiel 13

2-Isopropyl-5(6)-(5-methyl-3-oxo-4,5-dihydro-2H-6-pyridazinyl)-benzimidazol-hydrochlorid

Hergestellt analog Beispiel 11 aus 6,2 g 5-Methyl-6-(3-amino-4-isobutyrylamino-phenyl)-3-oxo-4,5-dihydro-2H-pyridazin.
Ausbeute : 4,7 g (71 % der Theorie),
Schmelzpunkt : 270-272 °C.

Beispiel 14

2-n-Pentyl-5(6)-(5-methyl-3-oxo-4,5-dihydro-2H-6-pyridazinyl)-benzimidazol-hydrochlorid

Hergestellt analog Beispiel 11 aus 1,8 g 5-Methyl-6-(3-amino-4-caproylamino-phenyl)-3-oxo-4,5-dihydro-2H-pyridazin.
Ausbeute : 0,8 g (41,8 % der Theorie),
Schmelzpunkt : 244 °C.

Beispiel 15

2-(4-Chlor-phenyl)-5(6)-(5-methyl-3-oxo-4,5-dihydro-2H-6-pyridazinyl)-benzimidazol

Hergestellt analog Beispiel 2b aus 1,45 g 2-(4-Chlor-phenyl)-5(6)-(1-oxo-2-methyl-3-methoxycarbonyl-1-propyl)-benzimidazol-hydrochlorid.
Ausbeute : 0,32 g (23 % der Theorie),
Schmelzpunkt : sintert ab 78 °C.

Beispiel 16

2-Trifluormethyl-5(6)-(-5-methyl-3-oxo-4,5-dihydro-2H-6-pyridazinvl)-benzimidazol

11 g 5-Methyl-6-(3,4-diamino-phenyl)-3-oxo-4,5-dihydro-2H-pyridazin werden mit 70 ml Trifluoressigsäure 2 Stunden zum Rückfluß erhitzt. Danach wird im Vakuum zur Trockne gebracht, mit Eiswasser versetzt und ammoniakalisch gestellt. Die Mischung wird mit Essigester extrahiert, die Essigesterphasen eingedampft und der Rückstand aus 50 %igem Äthanol unter Zugabe von Aktivkohle umkristallisiert.
Ausbeute : 6,4 g (50 % der Theorie),
Schmelzpunkt : 278-280 °C.

Beispiel 17

2-Trifluormethyl-5(6)-(5-methyl-3-oxo-2H-6-pyridazinyl)-benzimidazol

Hergestellt analog Beispiel 4 aus 6 g 2-Trifluormethyl-5(6)-(5-methyl-3-oxo-4,5-dihydro-2H-6-pyridazinyl)-benzimidazol.
Ausbeute : 0,8 g (13,5 % der Theorie),
Schmelzpunkt : über 300 °C (aus Äthanol).

Beispiel 18

5(6)-(5-Methyl-3-oxo-4,5-dihydro-2H-6-pyridazinyl)-benzimidazol-hydrochlorid

Hergestellt analog Beispiel 16 aus 2,0 g 5-Methyl-6-(3,4-diaminophenyl)-3-oxo-4,5-dihydro-2H-pyridazin und Ameisensäure. Das Hydrochlorid wird aus Methanol mit ätherischer Salzsäure gefällt und aus Äthanol/Äther umkristallisiert.
Ausbeute : 0,6 g (28,9 % der Theorie),
Schmelzpunkt : 300 °C (Zersetzung).

Beispiel 19

2-Mercapto-5(6)-(5-methyl-3-oxo-4,5-dihydro-2H-6-pyridazinyl)-benzimidazol

5,2 g Kaliumhydroxid werden in einer Mischung aus 50 ml Äthanol und 6,6 ml Wasser gelöst, dann 3,84 g Schwefelkohlenstoff und 11,0 g 5-Methyl-6-(3,4-diamino-phenyl)-3-oxo-4,5-dihydro-2H-pyridazin zugefügt und 3 Stunden zum Rückfluß erhitzt. Es wird mit Wasser verdünnt, mit Eisessig neutralisiert und das ausgefallene Produkt abgesaugt und mit Wasser gewaschen.

Ausbeute : 10,6 g Rohprodukt (94,3 % der Theorie),

Das Produkt wird über eine Kieselgelsäule gereinigt (Elutionsmittel : Chloroform/Äthanol = 9 : 1).

Schmelzpunkt : über 300 °C.

Beispiel 20

2-Methylmercapto-5(6)-(5-methyl-3-oxo-4,5-dihydro-2H-6-pyridazinyl)-benzimidazol

6,0 g des unter Beispiel 19 hergestellten Rohproduktes werden in 250 ml Dimethylformamid gelöst und anschließend 3,3 g Methyljodid und 1,4 g Natriumbicarbonat zugegeben. Die Mischung wird 1 Stunde bei Raumtemperatur und 1 Stunde bei 50 °C gerührt. Das Lösungsmittel wird im Vakuum abgedampft, der Rückstand mit Wasser verrieben und über eine Kieselgelsäule gereinigt.

Ausbeute : 1,5 g (23,8 % der Theorie),

Schmelzpunkt : 268 °C (Zersetzung).

Beispiel 21

2-Cyclohexyl-5(6)-(5-methyl-3-oxo-4,5-dihydro-2H-6-pyridazinyl)-benzimidazol-hydrochlorid

Hergestellt analog Beispiel 1c und 1d aus 9,7 g 5-Methyl-6-(3-nitro-4-cyclohexylcarbonylamino-phenyl)-3-oxo-4,5-dihydro-2H-pyridazin. Das Hydrochlorid wird aus Aceton mit ätherischer Salzsäure gefällt und aus Äthanol umkristallisiert.

Ausbeute : 2,3 g (24,7 % der Theorie)

Schmelzpunkt : 315-320 °C

Beispiel 22

2-(4-tert-Butyl-phenyl)-5(6)-(5-methyl-3-oxo-4,5-dihydro-2H-6-pyridazinyl)-benzimidazol-hydrochlorid-hydrat

a) 5-Methyl-6-[3-amino-4-(4-tert.butyl-benzoyl-amino)-phenyl]-3-oxo-4,5-dihydro-2H-pyridazin

Hergestellt analog Beispiel 1c aus 12,6 g 5-Methyl-6-[3-nitro-4-(4-tert.butyl-benzoyl-amino)-phenyl]-3-oxo-4,5-dihydro-2H-pyridazin. Nach dem Abfiltrieren des Katalysators wird das Produkt mit Wasser gefällt.

Ausbeute : 9,9 g (84,5 % der Theorie)

Schmelzpunkt : 215-217 °C.

b) 2-(4-tert.Butyl)-phenyl-5(6)-(5-methyl-3-oxo-4,5-dihydro-2H-6-pyridazinyl)-benzimidazol-hydrochlorid-hydrat

Hergestellt analog Beispiel 1d aus dem unter a) erhaltenen Produkt.

Ausbeute : 7,9 g (73 % der Theorie)

Schmelzpunkt : 294-298 °C.

Beispiel 23

2-(2-Pentyl)-5(6)-(5-methyl-3-oxo-4,5-dihydro-2H-6-pyridazinyl)-benzimidazol-hydrochlorid

a) 5-Methyl-6-[3-amino-4(-2-methyl-valeryl-amino)-phenyl]-3-oxo-4,5-dihydro-2H-pyridazin

Hergestellt analog Beispiel 22a) aus 12,5 g 5-Methyl-6-[3-nitro-4-(2-methyl-valeryl-amino)-phenyl]-3-oxo-4,5-dihydro-2H-pyridazin. Nach dem Versetzen mit Wasser wird das Produkt mit Essigester extrahiert.

Ausbeute : 4,2 g (36,6 % der Theorie)

Schmelzpunkt : 179-181 °C.

b) 2-(2-Pentyl)-5(6)-(5-methyl-3-oxo-4,5-dihydro-2H-6-pyridazinyl-benzimidazol)-hydrochlorid

Hergestellt analog Beispiel 1d aus dem unter a) erhaltenen Produkt.
Ausbeute : 1,3 g (28,4 % der Theorie)
Schmelzpunkt : sintert ab 150 °C.

Beispiel 24

2-(n-Hexyl)-5(6)-(5-methyl-3-oxo-4,5-dihydro-2H-6-pyridazinyl)-benzimidazol-hydrochlorid

a) 5-Methyl-6-(3-amino-4-heptanoyl-amino-phenyl)-3-oxo-4,5-dihydro-2H-pyridazin

Hergestellt analog Beispiel 22a) aus 11,75 g 5-Methyl-6-(3-nitro-4-heptanoyl-amino-phenyl)-3-oxo-4,5-dihydro-2H-pyridazin.
Ausbeute : 8,0 g (78 % der Theorie)
Schmelzpunkt : 158-160 °C.

b) 2-(n-Hexyl)-5(6)-(5-methyl-3-oxo-4,5-dihydro-2H-6-pyridazinyl)-benzimidazol-hydrochlorid

Hergestellt analog Beispiel 1d aus dem unter a) erhaltenen Produkt.
Ausbeute : 6,2 g (73,5 % der Theorie),
Schmelzpunkt : 225-227 °C.

Beispiel 25

1-Cyclohexyl-2-methyl-5(6)-(5-methyl-3-oxo-4,5-dihydro-2H-6-pyridazinyl)-benzimidazol

1,3 g 5-Methyl-6-(3-amino-4-cyclohexylamino-phenyl)-3-oxo-4,5-dihydro-2H-pyridazin werden mit 50 ml Eisessig 6 Stunden zum Rückfluß erhitzt. Das Reaktionsgemisch wird auf Eis gegossen, mit Ammoniak neutralisiert und das ausgefallene Produkt über eine Kieselgelsäule gereinigt (Elutionsmittel : Methylenchlorid/Aceton = 10 : 0 bis 9 : 1).
Ausbeute : 0,4 g (28,5 % der Theorie),
Schmelzpunkt : 259-262 °C (Zersetzung).

Beispiel 26

2-Hydroxy-5(6)-(5-methyl-3-oxo-4,5-dihydro-2H-6-pyridazinyl)-benzimidazol

2 g 2-Methylmercapto-5(6)-(5-methyl-3-oxo-4,5-dihydro-2H-6-pyridazinyl)-benzimidazol werden in 100 ml Eisessig gelöst, mit 10 ml 30 %igem Wasserstoffperoxid versetzt und 6 Stunden auf 50 °C erhitzt. Nach 3 und 4 Stunden werden jeweils noch 2 ml Wasserstoffperoxid nachgegeben. Das Reaktionsgemisch wird im Vakuum weitgehend eingeengt, auf Wasser gegossen und der Niederschlag aus Äthanol/Cyclohexan umkristallisiert.
Ausbeute : 0,37 g (21 % der Theorie),
Schmelzpunkt : über 300 °C.

Beispiel 27

2-(2-Methoxy-4-methylmercapto-phenyl)-5(6)-(5-methyl-3-oxo-4,5-dihydro-2H-6-pyridazinyl)-benzimidazol

2,5 g 5-Methyl-6-[3-amino-4-(2-methoxy-4-methylmercapto-benzoylamino)-phenyl]-3-oxo-4,5-dihydro-2H-pyridazin werden in 100 ml Eisessig 2,5 Stunden zum Rückfluß erhitzt, das Reaktionsgemisch heiß filtriert und nach dem Abkühlen in eine Mischung aus Eis und konzentriertem Ammoniak eingerührt. Das ausgefallene Produkt wird nach Trocknung aus Methylenchlorid umkristallisiert.
Ausbeute : 1,1 g (57,7 % der Theorie),
Schmelzpunkt : 155-165 °C (Zersetzung).

Beispiel 28

1-Cyclopropyl-2-methyl-5(6)-(5-methyl-3-oxo-4,5-dihydro-2H-6-pyridazinyl)-benzimidazol

a) 5-Methyl-6-(3-amino-4-cyclopropylamino-phenyl)-3-oxo-4,5-dihydro-2H-pyridazin

5,8 g 5-Methyl-6-(3-nitro-4-cyclopropylamino-phenyl)-3-oxo-4,5-dihydro-2H-pyridazin werden in einer

Mischung aus 100 ml Äthanol und 20 ml Eisessig in Gegenwart von 0,5 g 10 %iger Palladium-Kohle mit Wasserstoff von 5 at hydriert. Es werden 120 ml Eisessig zugefügt, der Katalysator abfiltriert und das Filtrat im Vakuum eingedampft. Der Rückstand wird ohne weitere Reinigung weiter umgesetzt.

b) 1-Cyclopropyl-2-methyl-5(6)-(5-methyl-3-oxo-4,5-dihydro-2H-6-pyridazinyl)-benzimidazol

Hergestellt analog Beispiel 25 aus dem unter a) erhaltenen Produkt und 30 ml Eisessig (Erhitzungsdauer : 3 Stunden, Säulenreinigung nicht erforderlich).
Ausbeute : 4,6 g (81,5 % der Theorie),
Schmelzpunkt : 246-250 °C.

Beispiel 29

2-Hexylmercapto-5(6)-(5-methyl-3-oxo-4,5-dihydro-2H-6-pyridazinyl)-benzimidazol

Hergestellt analog Beispiel 20 aus 2,6 g 2-Mercapto-5(6)-(5-methyl-3-oxo-4,5-dihydro-2H-6-pyridazinyl)-benzimidazol und 1,7 g 1-Bromhexan (Reaktionstemperatur : 80 °C, Reaktionszeit : 7 Stunden).
Ausbeute : 1,0 g (29 % der Theorie),
Schmelzpunkt : 172 °C (Zersetzung, aus Isopropanol/Petroläther).

Beispiel 30

1-Methyl-2-(4-methoxy-phenyl)-5-(5-methyl-3-oxo-4,5-dihydro-2H-6-pyridazinyl)-benzimidazol

6,4 g 5-Methyl-6-[3-nitro-4-(N-methyl-(4-methoxy-benzoyl)-amino)-phenyl]-4,5-dihydro-2H-pyridazin-3-on werden in 100 ml Äthanol in Gegenwart von 0,6 g 10 %iger Palladiumkohle bei Raumtemperatur 2,5 Stunden mit Wasserstoff von 5 bar behandelt. Der Katalysator wird abgesaugt und das Filtrat im Vakuum eingedampft. Der Rückstand wird durch Chromatographie an Kieselgel (Elutionsmittel : Methylenchlorid/Äthanol 19 : 1 bis 9 : 1) gereinigt. Die entsprechenden Fraktionen werden eingeengt und das Produkt durch Verreiben mit Äther kristallisiert.
Ausbeute : 0,15 g (3 % der Theorie),
Schmelzpunkt : 248-249 °C.

Beispiel 31

1-Benzyl-2-methyl-5-(5-methyl-3-oxo-4,5-dihydro-2H-6-pyridazinyl)-benzimidazol

a) 5-Methyl-6-(3-amino-4-benzylamino-phenyl)-4,5-dihydro-2H-pyridazin-3-on

6,75 g 5-Methyl-6-(3-nitro-4-benzylamino-phenyl)-4,5-dihydro-2H-pyridazin-3-on werden in einer Mischung aus 100 ml Äthanol und 20 ml Eisessig mit 0,7 g 10 %iger Palladiumkohle als Katalysator bei Raumtemperatur 3,5 Stunden mit Wasserstoff von 5 bar behandelt. Der Katalysator wird abfiltriert, das Filtrat eingedampft und der Rückstand ohne weitere Reinigung in der nächsten Stufe verwendet.

b) 1-Benzyl-2-methyl-5-(5-methyl-3-oxo-4,5-dihydro-2H-6-pyridazinyl)-benzimidazol

Das unter a) erhaltene Produkt wird in 100 ml Eisessig aufgenommen und 2,5 Stunden zum Rückfluß erhitzt. Danach wird mit Eis versetzt, ammoniakalisch gestellt und das ausgefallene Produkt durch Chromatographie an Kieselgel gereinigt (Elutionsmittel : zunächst reines Methylenchlorid, dann steigende Zusätze von Aceton bis zu einem Mischungsverhältnis Methylenchlorid/Aceton = 10 : 3). Nach dem Abdampfen des Elutionsmittels kristallisiert das Produkt beim Verreiben mit Äther.
Ausbeute : 2,2 g (33 % der Theorie),
Schmelzpunkt : 279 °C.

Beispiel 32

1-Cyclohexyl-2-methyl-6-(5-methyl-3-oxo-4,5-dihydro-2H-6-pyridazinyl)-benzimidazol

Hergestellt aus 1,3 g 5-Methyl-6-(3-amino-4-cyclohexylaminophenyl)-4,5-dihydro-2H-pyridazin-3-on analog Beispiel 31. Nach Abtrennung des 1-Cyclohexyl-2-methyl-5-(5-methyl-3-oxo-4,5-dihydro-2H-6-pyridazinyl)-benzimidazols durch Chromatographie an Kieselgel (Elutionsmittel : Methylenchlorid/Aceton = 10 : 0 bis 9 : 1) konnte das Produkt in reiner Form erhalten werden.
Ausbeute : 0,1 g (7 % der Theorie),
Schmelzpunkt : 237 °C (Zersetzung).

## Beispiel 33

2-Isopropylmercapto-5(6)-(5-methyl-3-oxo-4,5-dihydro-2H-6-pyridazinyl)-benzimidazol

3,4 g 2-Mercapto-5(6)-(5-methyl-3-oxo-4,5-dihydro-2H-6-pyridazinyl)-benzimidazol werden in 50 ml Dimethylformamid gelöst, 1,62 g Isopropylbromid und 1,1 g Natriumbicarbonat zugegeben und 20 Stunden auf dem Dampfbad gerührt. Das Lösungsmittel wird in Vakuum abgedampft und der Rückstand durch Chromatographie an Kieselgel (Elutionsmittel : Chloroform/Äthanol 9 : 1) gereinigt.

Ausbeute : 0,45 g (11 % der Theorie),
Schmelzpunkt : 120-121 °C.

## Beispiel 34

2-(2-Methoxy-4-methylsulfinyl-phenyl)-5(6)-(5-methyl-3-oxo-4,5-dihydro-2H-6-pyridazinyl)-benzimidazol

0,9 g 2-(2-Methoxy-4-methylmercapto-phenyl)-5(6)-(5-methyl-3-oxo-4,5-dihydro-2H-6-pyridazinyl)-benzimidazol werden in 50 ml Eisessig gelöst und mit einer Lösung von 0,24 g 30 %igem Wasserstoffperoxid in 10 ml Eisessig versetzt. Man läßt das Reaktionsgemisch 22 Stunden bei Raumtemperatur stehen, gießt dann auf Eis, stellt ammoniakalisch und reinigt den gebildeten Niederschlag durch Chromatographie an Kieselgel (Elutionsmittel : Methylenchlorid/Äthanol 19 : 1). Nach dem Abdampfen des Elutionsmittels wird der Rückstand durch Verreiben mit einem Gemisch aus Aceton und Äther kristallisiert.

Ausbeute : 0,5 g (53 % der Theorie),
Schmelzpunkt : 173-180 °C.

## Beispiel 35

2-(1-Phenyl-2-propyl)-5(6)-(5-methyl-3-oxo-4,5-dihydro-2H-6-pyridazinyl)-benzimidazol-hydrochlorid

5,1 g 5-Methyl-6-[3-amino-4-(3-phenyl-isobutyryl-amino)-phenyl]-4,5-dihydro-2H-pyridazin-3-on werden in 100 ml Eisessig 1,5 Stunden zum Rückfluß erhitzt. Der Eisessig wird abdestilliert, der Rückstand in einem Gemisch aus Äthanol und Aceton aufgenommen und durch Zusatz von ätherischer Salzsäure das Hydrochlorid gefällt, das beim Verreiben mit einem Gemisch aus Äther und Äthanol durchkristallisiert.

Ausbeute : 2,4 g (45 % der Theorie),
Schmelzpunkt : 235-239 °C.

## Beispiel 36

2-Cycloheptyl-5(6)-(5-methyl-3-oxo-4,5-dihydro-2H-6-pyridazinyl)-benzimidazol-hydrochlorid

Hergestellt analog Beispiel 35 aus 5,5 g 5-Methyl-6-(3-amino-4-cycloheptylcarbonylamino-phenyl)-4,5-dihydro-2H-pyridazin-3-on.

Ausbeute : 4,2 g (73 % der Theorie),
Schmelzpunkt : 255-259 °C.

## Beispiel 37

2-(4-Hydroxyphenyl)-5(6)-(5-methyl-3-oxo-4,5-dihydro-2H-6-pyridazinyl)-benzimidazol-hydrochlorid

Hergestellt analog Beispiel 35 aus 6,3 g 5-Methyl-6-[3-amino-4-(4-hydroxy-benzoylamino)-phenyl]-4,5-dihydro-2H-pyridazin-3-on.

Ausbeute : 4,4 g (55 % der Theorie),
Schmelzpunkt : 350-354 °C.

## Beispiel 38

2-(4-n-Hexyloxy-phenyl)-5(6)-(5-methyl-3-oxo-4,5-dihydro-2H-6-pyridazinyl)-benzimidazol-hydrochlorid

Hergestellt analog Beispiel 35 aus 5,7 g 5-Methyl-6-[3-amino-4-(4-n-hexyloxy-benzoyl-amino)-phenyl]-4,5-dihydro-2H-pyridazin-3-on.

Ausbeute : 3,55 g (60 % der Theorie),
Schmelzpunkt : 278-282 °C.

Beispiel 39

2-(3,4,5-Trimethoxy-phenyl)-5(6)-(5-methyl-3-oxo-4,5-dihydro-2H-pyridazinyl)-benzimidazol

Hergestellt analog Beispiel 35 aus 14,9 g 5-Methyl-6-[3-amino-4-(3,4,5-trimethoxy-benzoylamino)-phenyl]-4,5-dihydro-2H-pyridazin-3-on. Der nach dem Abdampfen des Eisessigs verbleibende Rückstand wird mit siedendem Äthanol extrahiert. Nach Einengen des Äthanols und Verrühren mit Äther erhält man die Substanz in kristalliner Form.
Ausbeute : 9,2 g (65 % der Theorie),
Schmelzpunkt : 295-300 °C.

Beispiel 40

1-(3-Methyl-butyl)-2-methyl-5-(5-methyl-3-oxo-4,5-dihydro-2H-6-pyridazinyl)-benzimidazol

a) 5-Methyl-6-[3-amino-4-(N-acetyl-N-(3-methyl-butyl)-amino)-phenyl]-4,5-dihydro-2H-pyridazin-3-on

6 g 5-Methyl-6-[3-nitro-4-(N-acetyl-N-(3-methyl-butyl)-amino)-phenyl]-4,5-dihydro-2H-pyridazin-3-on werden in 150 ml Äthanol gelöst, mit 4 g Raney-Nickel und 3 ml 98 %igem Hydrazinhydrat versetzt und 30 Minuten bei Raumtemperatur gerührt. Der Katalysator wird abfiltriert, das Filtrat eingedampft und der Rückstand ohne weitere Reinigung weiterverarbeitet.

b) 1-(3-Methyl-butyl)-2-methyl-5-(5-methyl-3-oxo-4,5-dihydro-2H-6-pyridazinyl)-benzimidazol

Hergestellt aus dem unter a) erhaltenen Produkt analog Beispiel 35, Reinigung durch Chromatographie an Kieselgel (Elutionsmittel : Methylenchlorid/Äthanol = 9 : 1).
Ausbeute : 2,2 g (43 % der Theorie),
Schmelzpunkt : 247 °C.

Beispiel 41

1-Benzyl-2-trifluormethyl-5-(5-methyl-3-oxo-4,5-dihydro-2H-6-pyridazinyl)-benzimidazol

Hergestellt analog Beispiel 31 aus 5,2 g 5-Methyl-6-(3-nitro-4-benzylamino-phenyl)-4,5-dihydro-2H-pyridazin-3-on.
Ausbeute : 1,4 g (23 % der Theorie),
Schmelzpunkt : 210-212 °C.

Beispiel 42

2-Methyl-5(6)-(5-äthyl-3-oxo-4,5-dihydro-2H-6-pyridazinyl)-benzimidazol

Hergestellt analog Beispiel 31 aus 1,2 g 5-Äthyl-6-(3-nitro-4-amino-phenyl)-4,5-dihydro-2H-pyridazin-3-on.
Ausbeute : 0,5 g (41 % der Theorie),
Schmelzpunkt : 291-295 °C.

Beispiel 43

2-(4-Methoxy-phenyl)-5(6)-(5-äthyl-3-oxo-4,5-dihydro-2H-6-pyridazinyl)-benzimidazol-hydrochlorid

Hergestellt analog Beispiel 35 aus 3,6 g 5-Äthyl-6-[3-amino-4-(4-methoxy-benzoylamino)-phenyl]-4,5-dihydro-2H-pyridazin-3-on.
Ausbeute : 3,6 g (91 % der Theorie),
Schmelzpunkt : 178-198 °C.

Beispiel 44

2-(3-Methoxy-phenyl)-5(6)-(5-methyl-3-oxo-4,5-dihydro-2H-6-pyridazinyl)-benzimidazol

Hergestellt analog Beispiel 35 aus 2,2 g 5-Methyl-6-[3-amino-4-(3-methoxy-benzoylamino)-phenyl]-4,5-dihydro-2H-pyridazin-3-on durch Reinigung mittels Chromatographie an Kieselgel (Elutionsmittel : Methylenchlorid/Äthanol = 19 : 1).
Ausbeute : 0,72 g (34 % der Theorie),
Schmelzpunkt : 295-298 °C.

Beispiel 45

2-(2-Brom-5-methoxy-phenyl)-5(6)-(5-methyl-3-oxo-4,5-dihydro-2H-6-pyridazinyl)-benzimidazol

Hergestellt analog Beispiel 44 aus 2,8 g 5-Methyl-6-[3-amino-4-(2-brom-5-methoxy-benzoylamino)-phenyl]-4,5-dihydro-2-H-pyridazin-3-on.
Ausbeute : 0,75 g (28 % der Theorie),
Schmelzpunkt : sintert ab 70 °C.

Beispiel 46

2-Undecyl-5(6)-(5-methyl-3-oxo-4,5-dihydro-2H-6-pyridazinyl)-benzimidazol

a) 5-Methyl-6-(3-amino-4-undecancarbonylamino-phenyl)-4,5-dihydro-2H-pyridazin-3-on

10 g 5-Methyl-6-(3-nitro-4-undecancarbonylamino-phenyl)-4,5-dihydro-2H-pyridazin-3-on werden in 100 ml Dimethylformamid gelöst und bei Raumtemperatur in Gegenwart von 1 g 10 %iger Palladiumkohle 1,2 Stunden mit Wasserstoff von 5 bar behandelt. Nach Abfiltrieren das Katalysators gießt man auf Eiswasser, extrahiert mit Methylenchlorid und dampft die organische Phase ein. Der ölige Rückstand wird direkt weiterverarbeitet.

b) 2-Undecyl-5(6)-(5-methyl-3-oxo-4,5-dihydro-2H-6-pyridazinyl)-benzimidazol

Das unter a) erhaltene Produkt wird in 100 ml Eisessig gelöst und eine Stunde zum Rückfluß erhitzt. Man engt die Lösung auf das halbe Volumen ein, gießt auf Eis und stellt ammoniakalisch. Das ausgefallene Produkt wird aus Essigester umkristallisiert. Eine weitere Fraktion erhält man durch chromatographische Reinigung der Mutterlaugen (Kieselgel, Elutionsmittel : Methylenchlorid/Äthanol = 19 : 1).
Ausbeute : 7,7 g (87 % der Theorie),
Schmelzpunkt : 165-169 °C (Zersetzung).

Beispiel 47

2-Methyl-5(6)-(5-n-butyl-3-oxo-4,5-dihydro-2H-6-pyridazinyl)-benzimidazol

Hergestellt analog Beispiel 31 aus 4 g 5-n-Butyl-6-(3-nitro-4-amino-phenyl)-4,5-dihydro-2H-pyridazin-3-on.
Ausbeute : 1,7 g (43 % der Theorie),
Schmelzpunkt : 184-186 °C (Zersetzung).

Beispiel 48

2-(4-Methoxy-phenyl)-5(6)-(5-n-butyl-3-oxo-4,5-dihydro-2H-6-pyridazinyl)-benzimidazol-hydrochlorid

Hergestellt analog Beispiel 44 aus 4,2 g 5-n-Butyl-6-[3-amino-4-(4-methoxy-benzoylamino)-phenyl]-4,5-dihydro-2H-pyridazin-3-on. Das Hydrochlorid wurde aus Essigester mit ätherischer Salzsäure gefällt.
Ausbeute : 3,2 g (73 % der Theorie),
Schmelzpunkt : 160-190 °C.

Beispiel 49

2-(1-Phenyl-1-äthyl)-5(6)-(5-methyl-3-oxo-4,5-dihydro-2H-6-pyridazinyl)-benzimidazol-hydrochlorid

Hergestellt analog Beispiel 35 aus 3 g 5-Methyl-6-[3-amino-4-(2-phenyl-propionylamino)-phenyl]-4,5-dihydro-2H-pyridazin-3-on.
Ausbeute : 1,6 g (51 % der Theorie),
Schmelzpunkt : sintert ab 85 °C.

Beispiel 50

2-Äthoxy-5(6)-(5-methyl-3-oxo-4,5-dihydro-2H-6-pyridazinyl)-benzimidazol-hydrochlorid

10,9 g 5-Methyl-6-(3,4-diamino-phenyl)-4,5-dihydro-2H-pyridazin-3-on werden in 12,5 g Orthokohlensäure-tetraäthylester suspendiert, mit 10 ml Dimethylsulfoxid versetzt und 1,5 Stunden zum Rückfluß erhitzt. Das Lösungsmittel wird abdestilliert und der ölige Rückstand durch Verrühren mit

Äther/Chloroform kristallisiert. Das Hydrochlorid wird aus Aceton/Äthanol 1 : 1 mit ätherischer Salzsäure gefällt und aus Äthanol/Isopropanol umkristallisiert.
Ausbeute : 7,6 g (49 % der Theorie),
Schmelzpunkt : über 300 °C.

Beispiel 51

2-(4-Methoxy-phenyl)-5(6)-(5-methyl-3-oxo-4,5-dihydro-2H-6-pyridazinyl)-benzimidazol-hydrochlorid

1,1 g 2-(4-Hydroxy-phenyl)-5-(5-methyl-3-oxo-4,5-dihydro-2H-6-pyridazinyl)-benzimidazol-hydrochlorid werden in 50 ml Methanol suspendiert und 0,34 g Natriummethylat zugegeben. Nachdem sich eine klare Lösung gebildet hat, fügt man portionsweise 2,52 g Dimethylsulfat und weitere 0,34 g Natriummethylat zu. Die gesamte Reaktionszeit beträgt 6 Stunden bei Raumtemperatur. Das Reaktionsgemisch wird auf Eis gegossen, neutralisiert und der Niederschlag über eine Kieselgelsäule gereinigt (Elutionsmittel : Methylenchlorid/Äthanol 50 : 1 bis 25 : 1). Das Hydrochlorid wird aus Methanol mit ätherischer Salzsäure gefällt.
Ausbeute : 0,59 g (52 % der Theorie),
Schmelzpunkt : 311 °C (Zers.)

Beispiel 52

2-(4-Methoxy-phenyl)-5(6)-(5-methyl-3-oxo-4,5-dihydro-2H-6-pyridazinyl)-benzimidazol-hydrochlorid

a) 5-Methyl-6-[3-amino-4-(4-methoxy-benzoylamino)-phenyl]-4,5-dihydro-2H-pyridazin-3-on

203 g 5-Methyl-6-[3-nitro-4-(4-methoxy-benzoylamino)-phenyl]-4,5-dihydro-2H-pyridazin-3-on werden in 1,7 l Dimethylformamid gelöst und in Gegenwart von 20 g 10 %iger Palladiumkohle 2 Stunden mit Wasserstoff von 5 bar behandelt. Nach Abfiltrieren des Katalysators gießt man das Filtrat auf 7,5 l Eiswasser und trocknet das ausgefallene Produkt bei 120 °C.
Ausbeute : 182 g (97 % der Theorie),
Schmelzpunkt : 253 °C.

b) 2-(4-Methoxy-phenyl)-5(6)-(5-methyl-3-oxo-4,5-dihydro-2H-6-pyridazinyl)-benzimidazol-hydrochlorid

180 g 5-Methyl-6-[3-amino-4-(4-methoxy-benzoylamino)-phenyl]-4,5-dihydro-2H-pyridazin-3-on werden in 1,5 l Eisessig suspendiert und 30 Minuten zum Rückfluß erhitzt, worauf 350 ml des Lösungsmittels unter vermindertem Druck abdestilliert werden. Das restliche Gemisch wird bei 70 °C mit Aktivkohle behandelt, abfiltriert und das Filtrat nach Abkühlen auf 20 °C unter Rühren in ein Gemisch aus 2 l Eiswasser und 1,5 l konzentriertem Ammoniak getropft. Durch Zugabe von weiterem Eis wird die Temperatur unter 20 °C gehalten. Das ausgefallene Produkt wird mit Wasser gewaschen und bei 80 °C getrocknet. Die Base wird durch Auflösen in Isopropanol, Versetzen mit konzentrierter Salzsäure und Verdünnen mit 2 l Aceton in das Hydrochlorid überführt. Die noch in den Mutterlaugen befindliche freie Base wird entsprechend behandelt.
Ausbeute : 176 g (93 % der Theorie),
Schmelzpunkt : 300-306 °C (Zersetzung).

Beispiel 53

2-(4-Methoxy-phenyl)-5(6)-(5-methyl-3-oxo-4,5-dihydro-2H-6-pyridazinyl)-benzimidazol-hydrochlorid

0,22 g 5-Methyl-6-(3,4-diamino-phenyl)-4,5-dihydro-2H-pyridazin-3-on und 0,2 g 4-Methoxy-benzamidin-hydrochlorid werden miteinander verrieben und dann 40 Minuten auf 160 °C erhitzt. Das Reaktionsgemisch wird durch Chromatographie and Kieselgel gereinigt (Elutionsmittel : Methylenchlorid/Äthanol 50 : 1 bis 25 : 1) und das Hydrochlorid aus Methanol mit ätherischer Salzsäure gefällt.
Ausbeute : 0,04 g (10 % der Theorie),
Schmelzpunkt : 305-307 °C (Zersetzung).

Beispiel 54

2-(4-Methoxy-phenyl)-5(6)-(5-methyl-3-oxo-4,5-dihydro-2H-6-pyridazinyl)-benzimidazol-hydrochlorid

Eine Mischung aus 0,22 g 5-Methyl-6-(3,4-diamino-phenyl)-4,5-dihydro-2H-pyridazin-3-on, 0,15 g 4-Methoxy-benzonitril und 0,17 g p-Toluolsulfonsäure werden 30 Minuten auf 160 °C erhitzt. Das Reaktionsgemisch wird wie in Beispiel 32 aufgearbeitet.

Ausbeute : 0,06 g (16 % der Theorie),
Schmelzpunkt : 304-309 °C (Zersetzung).

Beispiel 55

2-(4-Methoxy-phenyl)-5(6)-(5-methyl-3-oxo-4,5-dihydro-2H-6-pyridazinyl)-benzimidazol-hydrochlorid

Hergestellt analog Beispiel 54 mit 0,22 g Kaliumtertiärbutylat statt p-Toluolsulfonsäure.
Ausbeute : 0,03 g (8 % der Theorie),
Schmelzpunkt : 305-306 °C (Zersetzung).

Beispiel 56

2-(4-Methoxy-phenyl)-5(6)-(5-methyl-3-oxo-4,5-dihydro-2H-6-pyridazinyl)-benzimidazol-hydrochlorid

0,22 g 5-Methyl-6-(3,4-diamino-phenyl)-4,5-dihydro-2H-pyridazin-3-on und 0,42 g S-Methyl-4-methoxy-thiobenzoesäure-morpholidjodid werden in 3 ml Äthylenglykol 10 Minuten auf 140 °C erhitzt. Nach dem Abkühlen wird auf Eiswasser gegossen, das Produkt in Äther aufgenommen und wie in Beispiel 53 gereinigt.
Ausbeute : 0,13 g (35 % der Theorie),
Schmelzpunkt : 309-311 °C (Zersetzung).

Beispiel 57

2-(4-Methoxy-phenyl)-5(6)-(5-methyl-3-oxo-4,5-dihydro-2H-6-pyridazinyl)-benzimidazol-hydrochlorid

0,22 g 5-Methyl-6-(3,4-diamino-phenyl)-4,5-dihydro-2H-pyridazin-3-on und 0,29 g 4-Methoxy-benzoesäure-phenylester werden 30 Minuten auf 150 °C erhitzt. Das Reaktionsgemisch wird wie in Beispiel 53 aufgearbeitet.
Ausbeute : 0,23 g (62 % der Theorie),
Schmelzpunkt : 108-111 °C (Zersetzung).

Beispiel A

Tabletten zu 100 mg 2-(4-Methoxy-phenyl)-5(6)-(5-methyl-3-oxo-4,5-dihydro-2H-6-pyridazinyl)-benzimidazol-hydrochlorid

Zusammensetzung :
1 Tablette enthält :

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Wirkstoff und milchzucker mit wäßriger Lösung des Polyvinylpyrrolidons gleichmäßig befeuchten.
Feuchtsiebung : 1,5 mm
Trocknen : Umlufttrockenschrank 50 °C
Trockensieben : 1 mm
Dem Granulat die restlichen Hilfsstoffe zumischen und Endmischung zu Tabletten verpressen.
Tablettengewicht : 175 mg
Stempel : 8 mm ∅

Beispiel B

Dragees zu 50 mg 1-(4-Methoxy-phenyl)-5(6)-(5-methyl-3-oxo-4,5-dihydro-2H-6-pyridazinyl)-benzimidazol-hydrochlorid

1 Drageekern enthält :

| | |
|---|---|
| Wirksubstanz | 50,0 mg |
| Maisstärke getr. | 20,0 mg |
| Lösliche Stärke | 2,0 mg |

| | |
|---|---|
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.
Feuchtsiebung : 1,0 mm
Trockensiebung : 1,0 mm
Trocknung : 50 °C im Umlufttrockenschrank
Granulat und restliche Hilfsstoffe mischen und zu Kernen verpressen.
Kerngewicht : 80 mg
Stempel : 6 mm
Wölbungsradius : 5 mm
Die fertigen Kerne werden auf übliche Weise mit einem Zuckerüberzug im Dragierkessel versehen.
Drageegewicht : 120 mg

Beispiel C

Suppositorien zu 75 mg 2-(4-Methoxy-phenyl)-5(6)-(5-methyl-3-oxo-4,5-dihydro-2H-6-pyridazinyl)-benzimidazol-hydrochlorid

1 Zäpfchen enthält :

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

Herstellungsverfahren :

Die Zäpfchenmasse wird geschmolzen. Bei 38 °C wird die gemahlene Wirksubstanz in der Schmelze homogen dispergiert. Es wird auf 35 °C abgekühlt und in vorgekühlte Suppositorienformen ausgegossen.
Zäpfchengewicht : 1,7 g

Beispiel D

Ampullen zu 50 mg 2-(4-Methoxy-phenyl)-5(6)-(5-methyl-3-oxo-4,5-dihydro-2H-6-pyridazinyl)-benzimidazol-hydrochlorid

1 Ampulle enthält :

| | |
|---|---|
| Wirksubstanz | 50,0 mg |
| Sorbit | 250,0 mg |
| Dest. Wasser ad | 5,0 ml |

Herstellungsverfahren :

Die Wirksubstanz und Sorbit werden in dest. Wasser gelöst, dann wird auf das angegebene Volumen aufgefüllt und sterilfiltriert.
Abfüllung : in Ampullen zu 5 ml
Sterilisation : 20 Minuten bei 120 °C

Beispiel E

Tropfen mit 25 mg pro 5 ml 2-(4-Methoxy-phenyl)-5(6)-(5-methyl-3-oxo-4,5-dihydro-2H-6-pyridazinyl)-benzimidazol-hydrochlorid

| | |
|---|---|
| Wirksubstanz | 5,0 g |
| p-Oxybenzoesäuremethylester | 0,035 g |
| p-Oxybenzoesäurepropylester | 0,015 g |
| Anisöl | 0,05 g |
| Menthol | 0,06 g |

| | |
|---|---|
| Saccharin-Natrium | 1,0 g |
| Glycerin | 10,0 g |
| Äthanol | 40,0 g |
| Dest. Wasser ad | 100,0 ml |

Herstellungsverfahren :

Die Benzoesäureester werden in Äthanol gelöst und anschließend das Anisöl und das Menthol zugegeben. Dann wird die Wirksubstanz ; Glycerin und Saccharin-Natrium in Wasser gelöst zugegeben. Die Lösung wird anschließend klar filtriert.

**Ansprüche** (für die Vertragsstaaten : BE, CH, DE, FR, GB, IT, LU, NL, SE)

1. Neue in 5- oder 6-Stellung durch einen Pyridazinonring substituierte Benzimidazole der allgemeinen Formel

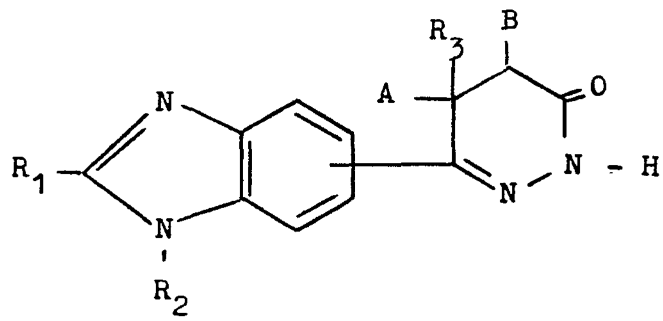

(I)

in der

A und B je ein Wasserstoffatom oder zusammen eine weitere Bindung,

$R_1$ ein Wasserstoffatom, die Trifluormethylgruppe, eine Alkylgruppe mit 1 bis 11 Kohlenstoffatomen, eine Cycloalkylgruppe mit 3 bis 7 Kohlenstoffatomen, eine gegebenenfalls durch eine Alkylgruppe mit 1 bis 6 Kohlenstoffatomen substituierte Hydroxy- oder Mercaptogruppe, eine Phenylalkylgruppe mit 1 bis 3 Kohlenstoffatomen im Alkylteil oder eine Phenylgruppe, die durch ein Halogenatom, eine Alkylgruppe mit 1 bis 4 Kohlenstoffatomen, eine Alkylsulfinylgruppe mit 1 bis 6 Kohlenstoffatomen und/oder eine gegebenenfalls durch eine Alkylgruppe mit 1 bis 6 Kohlenstoffatomen substituierte Hydroxy- oder Mercaptogruppe mono-, di- oder trisubstituiert sein kann, wobei die Substituenten des Phenylkerns gleich oder verschieden sein können,

$R_2$ ein Wasserstoffatom, eine Alkylgruppe mit 1 bis 5 Kohlenstoffatomen, eine Cycloalkylgruppe mit 3 bis 6 Kohlenstoffatomen oder eine Phenylakylgruppe mit 1 bis 3 Kohlenstoffatomen im Alkylteil und

$R_3$ eine Alkylgruppe mit 1 bis 6 Kohlenstoffatomen bedeuten, deren optisch aktive Antipoden, wenn A und B je ein Wasserstoffatom darstellen, und deren physiologisch verträgliche Säureadditionssalze mit anorganischen oder organischen Säuren.

2. Neue Benzimidazole der allgemeinen Formel I gemäß Anspruch 1,
in der

A und B je ein Wasserstoffatom oder zusammen eine weitere Bindung,

$R_1$ ein Wasserstoffatom, die Trifluormethylgruppe, eine Alkylgruppe mit 1 bis 11 Kohlenstoffatomen, eine Cycloalkylgruppe mit 3 bis 7 Kohlenstoffatomen, eine gegebenenfalls durch eine Alkylgruppe mit 1 bis 3 Kohlenstoffatomen substituierte Hydroxygruppe, eine gegebenenfalls durch eine Alkylgruppe mit 1 bis 6 Kohlenstoffatomen substituierte Mercaptogruppe, eine Phenylalkylgruppe mit 1 bis 3 Kohlenstoffatomen im Alkylteil, eine gegebenenfalls durch eine Hydroxygruppe, ein Halogenatom, eine Alkylgruppe mit 1 bis 4 Kohlenstoffatomen oder eine Alkoxygruppe mit 1 bis 6 Kohlenstoffatomen substituierte Phenylgruppe, eine durch eine Methoxygruppe und durch ein Halogenatom, eine Methylmercapto- oder Methylsulfinylgruppe substituierte Phenylgruppe oder eine durch 2 oder 3 Methoxygruppen substituierte Phenylgruppe,

$R_2$ ein Wasserstoffatom, eine Alkylgruppe mit 1 bis 5 Kohlenstoffatomen, eine Cyclopropyl-, Cyclohexyl- oder Benzylgruppe und

$R_3$ Eine Alkylgruppe mit 1 bis 4 Kohlenstoffatomen bedeuten, deren optisch aktiven Antipoden, wenn A und B je ein Wasserstoffatom darstellen, und deren physiologisch verträgliche Säureadditionssalze mit anorganischen oder organischen Säuren.

3. Neue Benzimidazole der allgemeinen Formel I gemäß Anspruch 1,

in der

A und B je ein Wasserstoffatom oder zusammen eine weitere Bindung,

$R_1$ ein Wasserstoffatom, die Methyl-, Isopropyl-, n-Pentyl-, 2-Pentyl-, n-Hexyl-, n-Undecyl-, Cyclopropyl-, Cyclohexyl-, Cycloheptyl-, Hydroxy-, Äthoxy-, Mercapto-, Methylmercapto-, Isopropylmercapto-, n-Hexylmercapto-, Trifluormethyl-, 1-Phenyläthyl-, 1-Phenyl-2-propyl-, Phenyl-, 2-Fluorophenyl-, 4-Hydroxyphenyl-, 2-Methoxyphenyl-, 3-Methoxyphenyl-, 4-Methoxyphenyl-, 2,4-Dimethoxyphenyl-, 3,4-Dimethoxyphenyl-, 3,4,5-Trimethoxyphenyl-, 4-n-Hexyloxyphenyl-, 4-Methylphenyl-, 4-tert.Butylphenyl-, 4-Chlorphenyl-, 2-Methoxy-4-methylmercaptophenyl-, 2-Methoxy-4-methylsulfinylphenyl- oder 2-Brom-5-methoxyphenylgruppe,

$R_2$ ein Wasserstoffatom, die Methyl-, 3-Methylbutyl-, Cyclopropyl-, Cyclohexyl- oder Benzylgruppe und

$R_3$ die Methyl-, Äthyl- oder n-Butylgruppe bedeuten, deren optisch aktive Antipoden, wenn A und B je ein Wasserstoffatom darstellen, und deren physiologisch verträgliche Säureadditionssalze mit anorganischen oder organischen Säuren.

4. Neue Benzimidazole der allgemeinen Formel I gemäß Anspruch 1,
in der

A und B je ein Wasserstoffatom oder zusammen eine weitere Bindung,

$R_1$ ein Wasserstoffatom, die Methyl-, 2-Pentyl-, 1-Phenyläthyl-, Äthoxy-, Methylmercapto-, 4-Methylphenyl-, 2-Fluorphenyl-, 4-Methoxyphenyl-, 4-Hexyloxyphenyl- oder 2,4-Dimethoxyphenylgruppe,

$R_2$ ein Wasserstoffatom, die Methyl- oder Benzylgruppe und

$R_3$ die Methylgruppe bedeuten, deren optisch aktive Antipoden, wenn A und B je ein Wasserstoffatom darstellen, und deren physiologisch verträgliche Säureadditionssalze mit anorganischen oder organischen Säuren.

5. Neue Benzimidazole der allgemeinen Formel I gemäß Anspruch 1,
in der

$R_1$, $R_2$ und $R_3$ wie im Anspruch 2 definiert sind und

A und B je ein Wasserstoffatom bedeuten, deren optisch aktive Antipoden und deren physiologisch verträgliche Säureadditionssalze mit anorganischen oder organischen Säuren.

6. 2-(4-Methoxy-phenyl)-5(6)-(5-methyl-3-oxo-4,5-dihydro-2H-6-pyridazinyl)-benzimidazol, dessen optisch aktive Antipoden und Säureadditionssalze.

7. 1-Benzyl-2-methyl-5-(5-methyl-3-oxo-4,5-dihydro-2H-6-pyridazinyl)-benzimidazol, dessen optisch aktive Antipoden und Säureadditionssalze.

8. Benzimidazole gemäß den Ansprüchen 1 bis 7 zur Verwendung bei der Bekämpfung von cardiovasculären Erkrankungen, von Ulcera, von Viren und viralen Erkrankungen.

9. Arzneimittel, enthaltend eine Verbindung gemäß den Ansprüchen 1 bis 7 neben einem oder mehreren inerten Trägerstoffen und/oder Verdünnungsmitteln.

10. Verfahren zur Herstellung von neuen in 5- oder 6-Stellung durch einen Pyridazinonring substituierten Benzimidazolen der allgemeinen Formel I der Ansprüche 1 bis 7, dadurch gekennzeichnet, daß

a) eine gegebenenfalls im Reaktionsgemisch hergestellte Verbindung der allgemeinen Formel

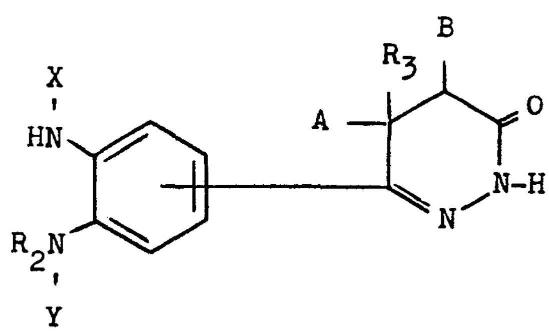

(II)

in der

A, B, $R_2$ und $R_3$ wie in den Ansprüchen 1 bis 7 definiert sind, einer der Reste X oder Y ein Wasserstoffatom und der andere der Reste X oder Y oder beide Reste X und Y je eine Gruppe der Formel

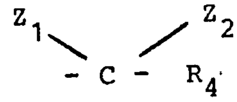

darstellen, in der

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen und

$R_4$ die für $R_1$ eingangs in den Ansprüchen 1 bis 7 erwähnten Bedeutungen besitzt oder eine gegebenenfalls durch eine Alkylgruppe mit 1 bis 3 Kohlenstoffatomen substituierte Aminogruppe darstellen, oder dessen Alkalisalze, wenn $R_4$ die Mercaptogruppe darstellt, cyclisiert wird oder

b) eine Carbonsäure der allgemeinen Formel

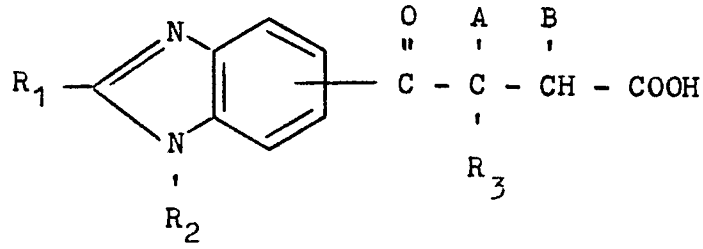

(III)

in der

A, B und $R_1$ bis $R_3$ wie in den Ansprüchen 1 bis 7 definiert sind, oder deren Ester, Amide oder Halogenide mit Hydrazin umgesetzt werden und gewünschtenfalls anschließend eine gemäß den Verfahren a oder b erhaltene Verbindung der allgemeinen Formel I, in der A und B je ein Wasserstoffatom darstellet, mittels Dehydrierung in eine Verbindung der allgemeinen Formel I, in der A und B zusammen eine weitere Bindung darstellen, übergeführt wird und/oder eine erhaltene Verbindung deß allgemeinen Formel I, in der $R_1$ die Hydroxy- oder Mercaptogruppe oder eine Phenylgruppe, die durch eine Hydroxy- und/oder Mercaptogruppe mono-, di- oder trisubstituiert ist, und/oder $R_2$ ein Wasserstoffatom darstellt, mittels Alkylierung in eine entsprechende Alkoxy-, Alkylmercapto- und/oder Alkylverbindung der allgemeinen Formel I übergeführt wird und/oder eine erhaltene Verbindung der allgemeinen Formel I, in der $R_1$ eine durch mindestens eine Alkylmercaptogruppe substituierte Phenylgruppe darstellt, mittels Oxidation in eine entsprechende Alkylsulfinylphenylverbindung der allgemeinen Formel I übergeführt wird und/oder eine erhaltene Verbindung der allgemeinen Formel I, in der $R_1$ eine Alkylmercaptogruppe darstellt, mittels Oxidation und anschließende hydrolytische Abspaltung der gebildeten Alkylsulfinyl- bzw. Alkylsulfonylgruppe in eine entsprechende Hydroxyverbindung der allgemeinen Formel I übergeführt wird und/oder ein erhaltenes racemisches Gemisch einer Verbindung der allgemeinen Formel I, wenn A und B je ein Wasserstoffatom darstellen, mittels Racematspaltung in ihre optisch aktiven Antipoden aufgespalten wird und/oder eine erhaltene Verbindung der allgemeinen Formel I in ihre physiologisch verträglichen Säureadditionssalze mit anorganischen oder organischen Säuren übergeführt wird.

**Ansprüche** (für den Vertragsstaat : AT)

1. Verfahren zur Herstellung von neuen in 5- oder 6-Stellung durch einen Pyridazinonring substituierten Benzimidazolen der allgemeinen Formel

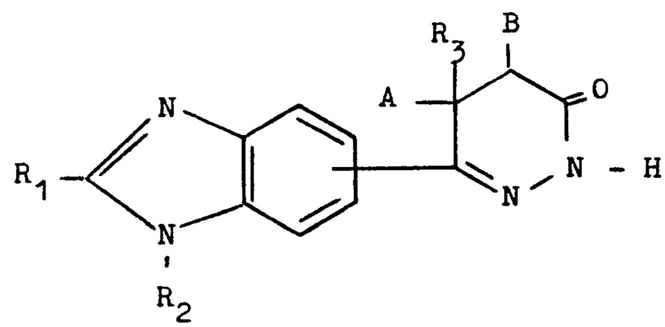

(I)

in der

A und B je ein Wasserstoffatom oder zusammen eine weitere Bindung,

$R_1$ ein Wasserstoffatom, die Trifluormethylgruppe, eine Alkylgruppe mit 1 bis 11 Kohlenstoffatomen, eine Cycloalkylgruppe mit 3 bis 7 Kohlenstoffatomen, eine gegebenenfalls durch eine Alkylgruppe mit 1 bis 6 Kohlenstoffatomen substituierte Hydroxy- oder Mercaptogruppe, eine Phenylalkylgruppe mit 1 bis 3 Kohlenstoffatomen im Alkylteil oder eine Phenylgruppe, die durch ein Halogenatom, eine Alkylgruppe mit 1 bis 4 Kohlenstoffatomen, eine Alkylsulfinylgruppe mit 1 bis 6 Kohlenstoffatomen und/oder eine gegebenenfalls durch eine Alkylgruppe mit 1 bis 6 Kohlenstoffatomen substituierte Hydroxy- oder

Mercaptogruppe mono-, di- oder trisubstituiert sein kann, wobei die Substituenten des Phenylkerns gleich oder verschieden sein können,

$R_2$ ein Wasserstoffatom, eine Alkylgruppe mit 1 bis 5 Kohlenstoffatomen, eine Cycloalkylgruppe mit 3 bis 6 Kohlenstoffatomen oder eine Phenylalkylgruppe mit 1 bis 3 Kohlenstoffatomen im Alkylteil und

$R_3$ eine Alkylgruppe mit 1 bis 6 Kohlenstoffatomen bedeuten, sowie von deren optisch aktiven Antipoden, wenn A und B je ein Wasserstoffatom darstellen, und von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

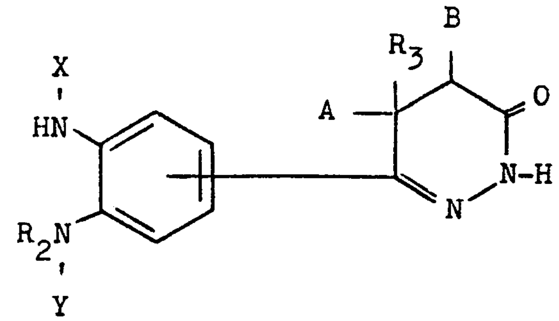

(II)

in der

A, B, $R_2$ und $R_3$ wie eingangs definiert sind, einer der Reste X oder Y ein Wasserstoffatom und der andere der Reste X oder Y oder beide Reste X und Y je eine Gruppe der Formel

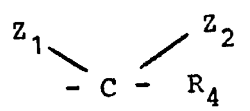

darstellen, in der

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen und

$R_4$ die für $R_1$ eingangs erwähnten Bedeutungen besitzt oder eine gegebenenfalls durch eine Alkylgruppe mit 1 bis 3 Kohlenstoffatomen substituierte Aminogruppe darstellen, oder dessen Alkalisalze, wenn $R_4$ die Mercaptogruppe darstellt, cyclisiert wird oder

b) eine Carbonsäure der allgemeinen Formel

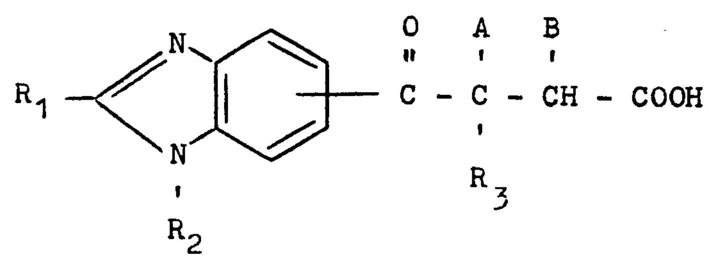

(III)

in der

A, B und $R_1$ bis $R_3$ wie eingangs definiert sind, oder deren Ester, Amide oder Halogenide mit Hydrazin umgesetzt wird und gewünschtenfalls anschließend eine gemäß den Verfahren a) oder b) erhaltene Verbindung der allgemeinen Formel (I), in der A und B je ein Wasserstoffatom darstellt, mittels Dehydrierung in eine Verbindung der allgemeinen Formel (I), in der A und B zusammen eine weitere Bindung darstellen, übergeführt wird und/oder eine erhaltene Verbindung der allgemeinen Formel (I), in der $R_1$ die Hydroxy- oder Mercaptogruppe oder eine Phenylgruppe, die durch eine Hydroxy- und/oder Mercaptogruppe mono-, di- oder trisubstituiert ist, und/oder $R_2$ ein Wasserstoffatom darstellt, mittels Alkylierung in eine entsprechende Alkoxy-, Alkylmercapto- und/oder Alkylverbindung der allgemeinen Formel (I) übergeführt wird und/oder eine erhaltene Verbindung der allgemeinen Formel (I), in der $R_1$ eine durch mindestens eine Alkylmercaptogruppe substituierte Phenylgruppe darstellt, mittels Oxidation in eine entsprechende Alkylsulfinylphenylverbindung der allgemeinen Formel (I) übergeführt wird und/oder eine erhaltene Verbindung der allgemeinen Formel (I), in der $R_1$ eine Alkylmercaptogruppe darstellt, mittels Oxidation und anschließende hydrolytische Abspaltung der gebildeten Alkylsulfinyl- bzw.

Alkylsulfonylgruppe in eine entsprechende Hydroxyverbindung der allgemeinen Formel (I) übergeführt wird und/oder ein erhaltenes racemisches Gemisch einer Verbindung der allgemeinen Formel (I), wenn A und B je ein Wasserstoffatom darstellen, mittels Racematspaltung in ihre optisch aktiven Antipoden aufgespalten wird und/oder eine erhaltene Verbindung der allgemeinen Formel (I) in ihre physiologisch verträglichen Säureadditionssalze mit anorganischen oder organischen Säuren übergeführt wird.

2. Verfahren gemäß Anspruch 1 zur Herstellung von neuen in 5-Stellung durch einen Pyridazinonring substituierten Benzimidazolen der allgemeinen Formel

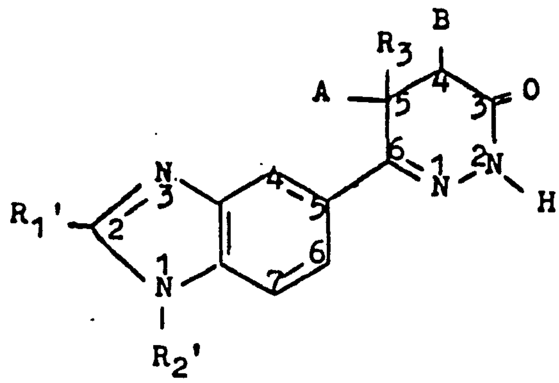

(Ia)

in der

$R_3$, A und B wie im Anspruch 1 definiert sind,

$R_1'$ ein Wasserstoffatom, die Trifluormethylgruppe, eine Alkylgruppe mit 1 bis 6 Kohlenstoffatomen, eine Cycloalkylgruppe mit 3 bis 7 Kohlenstoffatomen, eine gegebenenfalls durch eine Alkylgruppe mit 1 bis 6 Kohlenstoffatomen substituierte Hydroxy- oder Mercaptogruppe oder eine Phenylgruppe, die durch ein Halogenatom, eine Alkylgruppe mit 1 bis 4 Kohlenstoffatomen und/oder eine gegebenenfalls durch eine Alkylgruppe mit 1 bis 3 Kohlenstoffatomen substituierte Hydroxy- oder Mercaptogruppe mono-, di- oder trisubstituiert sein kann, wobei die Substituenten des Phenylkerns gleich oder verschieden sein können, und

$R_2'$ ein Wasserstoffatom, eine Alkylgruppe mit 1 bis 3 Kohlenstoffatomen oder eine Cycloalkylgruppe mit 3 bis 6 Kohlenstoffatomen bedeuten, sowie von deren optisch aktiven Antipoden, wenn A und B je ein Wasserstoffatom darstellen, von deren 1 H und 3 H Tautomeren und von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

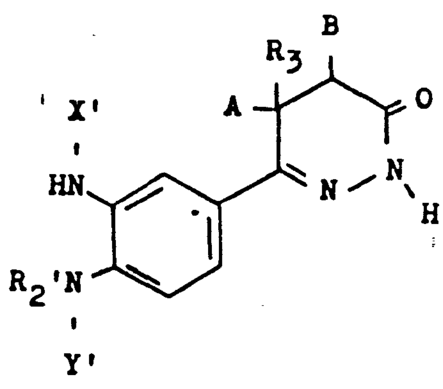

(IIa)

in der

A, B, $R_2'$ und $R_3$ wie eingangs definiert sind, einer der Reste X' oder Y' ein Wasserstoffatom und der andere der Reste X' oder Y' eine Gruppe der Formel

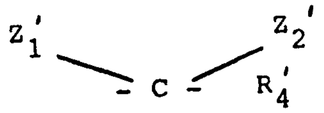

darstellt, in der

$Z_1'$ und $Z_2'$, die gleich oder verschieden sein können, gegebenenfalls durch niedere Alkylgruppen substituierte Hydroxy- oder Mercaptogruppen oder

$Z_1'$ und $Z_2'$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen und

$R_4'$ die für $R_1'$ eingangs erwähnten Bedeutungen besitzt oder eine gegebenenfalls durch eine Alkylgruppe mit 1 bis 3 Kohlenstoffatomen substituierte Aminogruppe darstellen, oder dessen Alkalisalze, wenn $R_4'$ die Mercaptogruppe darstellt, cyclisiert wird oder

b) eine Carbonsäure der allgemeinen Formel

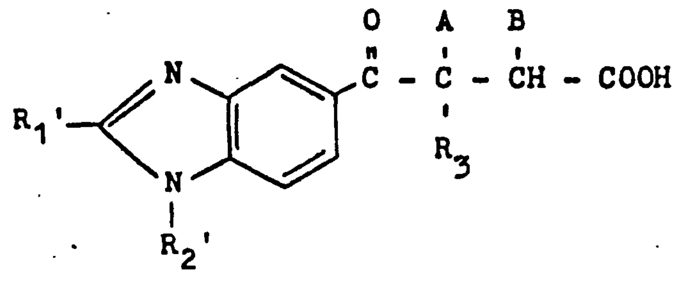

(IIIa)

in der

A, B, $R_1'$, $R_2'$ und $R_3$ wie eingangs definiert sind, oder deren Ester, Amide oder Halogenide mit Hydrazin umgesetzt wird und gewünschtenfalls anschließend eine gemäß den Verfahren a) oder b) erhaltene Verbindung der allgemeinen Formel (Ia), in der A und B je ein Wasserstoffatom darstellt, mittels Dehydrierung in eine Verbindung der allgemeinen Formel (Ia), in der A und B zusammen eine weitere Bindung darstellen, übergeführt wird und/oder eine erhaltene Verbindung der allgemeinen Formel (Ia), in der $R_1'$ die Hydroxy- oder Mercaptogruppe oder eine Phenylgruppe, die durch eine Hydroxy- und/ oder Mercaptogruppe mono-, di- oder trisubstituiert ist, und/oder $R_2'$ ein Wasserstoffatom darstellt, mittels Alkylierung in eine entsprechende Alkoxy-, Alkylmercapto- und/oder Alkylverbindung der allgemeinen Formel (Ia) übergeführt wird und/oder eine erhaltene Verbindung der allgemeinen Formel (Ia), in der $R_1'$ eine Alkylmercaptogruppe darstellt, mittels Oxidation und anschließende hydrolytische Abspaltung der gebildeten Alkylsulfinyl- bzw. Alkylsulfonylgruppe in eine entsprechende Hydroxyverbindung der allgemeinen Formel (Ia) übergeführt wird und/oder ein erhaltenes racemisches Gemisch einer Verbindung der allgemeinen Formel (Ia) mittels Racematspaltung in ihre optisch aktiven Antipoden aufgespalten wird und/oder eine erhaltene Verbindung der allgemeinen Formel (Ia) in ihre physiologisch verträglichen Säureadditionssalze mit anorganischen oder organischen Säuren übergeführt wird.

Priorität : 25. 08. 1978 (P 28 37 161.1).

3. Verfahren gemäß Anspruch 1 zur Herstellung von neuen in 5- oder 6-Stellung durch einen Pyridazinonring substituierten Benzimidazolen der allgemeinen Formel

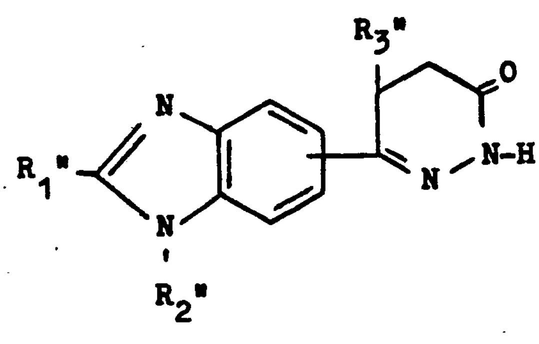

(Ib)

in der

$R_1''$ eine Methyl-, Trifluormethyl-, Cycloheptyl-, Äthoxy-, Isopropylmercapto-, Methoxyphenyl-, Hydroxyphenyl-, Trimethoxyphenyl- oder Brom-methoxyphenylgruppe, eine Phenylalkylgruppe mit 1 bis 3 Kohlenstoffatomen im Alkylteil, eine Alkylgruppe mit 7 bis 11 Kohlenstoffatomen, eine Alkoxyphenylgruppe mit 4 bis 6 Kohlenstoffatomen im Alkoxyteil oder eine durch Alkylsulfinylgruppen mit 1 bis 6 Kohlenstoffatomen im Alkylteil mono- oder disubstituierte Phenyl- oder Methoxyphenylgruppe,

$R_2''$ ein Wasserstoffatom, eine Methyl- oder Cyclohexylgruppe, eine Alkylgruppe mit 4 oder 5 Kohlenstoffatomen oder eine Phenylalkylgruppe mit 1 bis 3 Kohlenstoffatomen im Alkylteil und

$R_3''$ eine Methyl-, Äthyl- oder Butylgruppe bedeuten, und von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

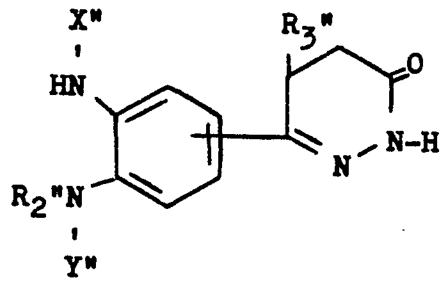

(IIb)

in der

$R_2''$ und $R_3''$ wie eingangs definiert sind, einer der Rexte X″ oder Y″ ein Wasserstoffatom und der andere der Reste X″ oder Y″ eine Gruppe der Formel

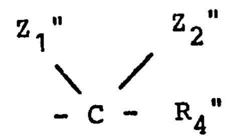

darstellt, in der

$Z_1''$ und $Z_2''$, die gleich oder verschieden sein können, gegebenenfalls substituierte Aminogruppen oder gegebenenfalls durch niedere Alkylgruppen substituierte Hydroxy- oder Mercaptogruppen oder

$Z_1''$ und $Z_2''$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen und

$R_4''$ die für $R_1''$ eingangs erwähnten Bedeutungen besitzt oder eine gegebenenfalls durch eine Alkylgruppe mit 1 bis 3 Kohlenstoffatomen substituierte Aminogruppe darstellen, oder dessen Alkalisalze, wenn $R_4''$ die Mercaptogruppe darstellt, cyclisiert wird oder

b) eine Carbonsäure der allgemeinen Formel

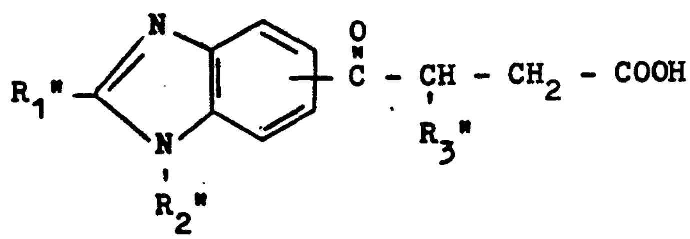

(IIIb)

in der

$R_1''$ bis $R_3''$ wie eingangs definiert sind, oder deren Ester, Amide oder Halogenide mit Hydrazin umgesetzt wird und gewünschtenfalls anschließend eine gemäß den Verfahren a) oder b) erhaltene Verbindung der allgemeinen Formel (Ib), in der $R_1''$ die Hydroxy- oder Mercaptogruppe oder eine Phenylgruppe, die durch eine Hydroxy- und/oder Mercaptogruppe mono-, di- oder trisubstituiert ist, und/oder $R_2''$ ein Wasserstoffatom darstellt, mittels Alkylierung in eine entsprechende Alkoxy-, Alkylmercapto- und/oder Alkylverbindung der allgemeinen Formel (Ib) übergeführt wird und/oder eine erhaltene Verbindung der allgemeinen Formel (Ib) in der $R_1''$ eine durch mindestens eine Alkylmercaptogruppe substituierte Phenylgruppe darstellt, mittels Oxidation in eine entsprechende Alkylsulfinylverbindung der allgemeinen Formel (Ib) übergeführt wird und/oder ein erhaltenes racemisches Gemisch einer Verbindung der allgemeinen Formel (Ib) mittels Racematspaltung in ihre optisch aktiven Antipoden aufgespalten wird und/oder eine erhaltene Verbindung der allgemeinen Formel (Ib) in ihre physiologisch verträglichen Säureadditionssalze mit anorganischen oder organischen Säuren übergeführt wird.

Priorität : 01.06.1979 (P 29 22 336.7)

4. Verfahren gemäß Anspruch 1, 2 und 3, dadurch gekennzeichnet, daß die Umsetzung in einem

Lösungsmittel und bei Temperaturen zwischen 0 und 250 °C durchgeführt wird.

5. Verfahren gemäß Anspruch 1a, 2a, 3a und 4, dadurch gekennzeichnet, daß die Cyclisierung in Gegenwart eines Kondensationsmittels oder in Gegenwart einer Base durchgeführt wird.

6. Verfahren gemäß Anspruch 1b, 2b und 3b, dadurch gekennzeichnet, daß die Umsetzung in einem Lösungsmittel bei Temperaturen zwischen 0 und 150 °C durchgeführt wird.

7. Verfahren gemäß Anspruch 1b, 2b, 3b und 6, dadurch gekennzeichnet, daß die Umsetzung in Gegenwart eines sauren Kondensationsmittels durchgeführt wird.

**Claims** (for the Contracting states : BE, CH, DE, FR, GB, IT, LU, NL, SE)

1. New benzimidazoles of general formula

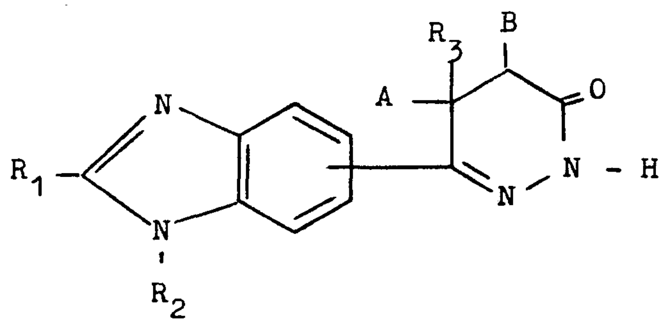

(I)

substituted in the 5 or 6 position by a pyridazinone ring,
wherein

A and B each represent a hydrogen atom or together represent another bond,

$R_1$ represents a hydrogen atom, the trifluoromethyl group, in alkyl group with 1 to 11 carbon atoms, a cycloalkyl group with 3 to 7 carbon atoms, a hydroxy or mercapto group optionally substituted by an alkyl group with 1 to 6 carbon atoms, a phenylalkyl group 1 to 3 carbon atoms in the alkyl part or a phenyl group, which may be mono-, di or trisubstituted by a halogen atom, an alkyl group with 1 to 4 carbon atoms, an alkylsulfinyl group with 1 to 6 carbon atoms and/or a hydroxy or mercapto group optionally substituted by an alkyl group with 1 to 6 carbon atoms, wherein the substituents of the phenyl nucleus may be the same or different,

$R_2$ represents a hydrogen atom, an alkyl group with 1 to 5 carbon atoms, a cycloalkyl group with 3 to 6 carbon atoms or a phenylalkyl group with 1 to 3 carbon atoms in the alkyl part and

$R_3$ represents an alkyl group with 1 to 6 carbon atoms, the optically active antipodes thereof, where A and B each represents a hydrogen atom, and the physiologically acceptable acid addition salts thereof with inorganic or organic acids.

2. New benzimidazoles of general formula I as claimed in Claim 1,
wherein

A and B each represent a hydrogen atom or together represent another bond,

$R_1$ represents a hydrogen atom, the trifluoromethyl group, an alkyl group with 1 to 11 carbon atoms, a cycloalkyl group with 3 to 7 carbon atoms, a hydroxy group optionally substituted by an alkyl group with 1 to 3 carbon atoms, a mercapto group optionally substituted by an alkyl group with 1 to 6 carbon atoms, a phenylalkyl group with 1 to 3 carbon atoms in the alkyl part ; a phenyl group optionally substituted by a hydroxy group, a halogen atom, an alkyl group with 1 to 4 carbon atoms or an alkoxy group with 1 to 6 carbon atoms, a phenyl group substituted by a methoxy group and a halogen atom, a methylmercapto or methylsulfinyl group or a phenyl group substituted by 2 or 3 methoxy groups,

$R_2$ represents a hydrogen atom, an alkyl group with 1 to 5 carbon atoms, a cyclopropyl, cyclohexyl or benzyl group and

$R_3$ represents an alkyl group with 1 to 4 carbon atoms, the optically active antipodes thereof, where A and B each represents a hydrogen atom, and the physiologically acceptable acid addition salts thereof with inorganic or organic acids.

3. New benzimidazoles of general formula I as claimed in Claim 1,
wherein

A and B each represents a hydrogen atom or together represent another bond

$R_1$ represents a hydrogen atom, the methyl, isopropyl, n-pentyl, 2-pentyl, n-hexyl, n-undecyl, cyclopropyl, cyclohexyl, cycloheptyl, hydroxy, ethoxy, mercapto, methylmercapto, isopropylmercapto, n-hexylmercapto, trifluoromethyl, 1-phenylethyl, 1-phenyl-2-propyl, phenyl, 2-fluorophenyl, 4-hydroxyphenyl, 2-methoxyphenyl, 3-methoxyphenyl, 4-methoxyphenyl, 2,4-dimethoxyphenyl, 3,4-dimethoxyphenyl, 3,4,5-trimethoxyphenyl, 4-n-hexyloxyphenyl, 4-methylphenyl, 4-tert. butylphenyl, 4-chlorophenyl, 2-methoxy-4-methylmercaptophenyl, 2-methoxy-4-methylsulfinylphenyl or 2-bromo-5-methoxyphenyl group,

$R_2$ represents a hydrogen atom, the methyl, 3-methylbutyl, cyclopropyl, cyclohexyl or benzyl group and

$R_3$ represents the methyl, ethyl or n-butyl group, the optically active antipodes thereof, where A and B each represents a hydrogen atom, and the physiologically acceptable acid addition salts thereof with inorganic or organic acids.

4. New benzimidazoles of general formula I as claimed in Claim 1, wherein

A and B each represents a hydrogen atom or together represent another bond,

$R_1$ represents a hydrogen atom, the methyl, 2-pentyl, 1-phenylethyl, ethoxy, methylmercapto, 4-methylphenyl, 2-fluorophenyl, 4-methoxyphenyl, 4-hexyloxyphenyl or 2,4-dimethoxyphenyl group,

$R_2$ represents a hydrogen atom, the methyl or benzyl group and

$R_3$ represents the methyl group, the optically active antipodes thereof, where A and B each represents a hydrogen atom, and the physiologically acceptable acid addition salts thereof with inorganic or organic acids.

5. New benzimidazoles of general formula I as claimed in Claim 1, wherein

$R_1$, $R_2$ and $R_3$ are defined as in Claim 2 and

A and B each represents a hydrogen atom, the optically active antipodes thereof and the physiologically acceptable acid addition salts thereof with inorganic or organic acids.

6. 2-(4-Methoxy-phenyl)-5(6)-(5-methyl-3-oxo-4,5-dihydro-2H-6-pyridazinyl)-benzimidazole, the optically active antipodes and acid addition salts thereof.

7. 1-Benzyl-2-methyl-5-(5-methyl-3-oxo-4,5-dihydro-2H-6-pyridazinyl)-benzimidazole, the optically active antipodes and acid addition salts thereof.

8. Benzimidazoles as claimed in Claims 1 to 7 for use in the treatment of cardiovascular diseases, ulcers, viruses and viral diseases.

9. Pharmaceutical compositions, containing a compound as claimed in Claims 1 to 7, in addition to one or more inert carriers and/or diluents.

10. A process for the preparation of new benzimidazoles of general formula (I) substituted in the 5 or 6 position by a pyridazinone ring, as claimed in Claims 1 to 7, characterised in that

a) a compound of general formula II,

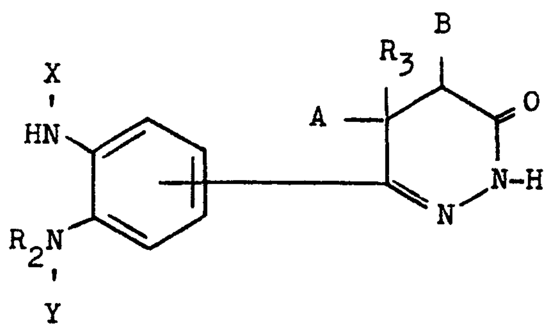

(II)

optionally prepared in the reaction mixture, wherein

A, B, $R_2$ and $R_3$ are defined as in claims 1 to 7, one of the radicals X or Y represents a hydrogen atom and the other of the radicals X or Y or both radicals X and Y each represents a group of formula

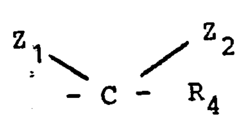

wherein

$Z_1$ and $Z_2$, which may be the same or different, represent optionally substituted amino groups or hydroxy or mercapto groups optionally substituted by a lower alkyl group, or

$Z_1$ and $Z_2$ together represent an oxygen or sulfur atom, an imino group optionally substituted by an alkyl group with 1 to 3 carbon atoms, an alkylenedioxy or alkylenedithio group, each with 2 or 3 carbon atoms and

$R_4$ has the meanings given for $R_1$ in claims 1 to 7, or represents an amino group optionally substituted by an alkyl group with 1 to 3 carbon atoms, or the alkali salts thereof, if $R_4$ represents the mercapto group, is cyclised, or

b) a carboxylic acid of general formula

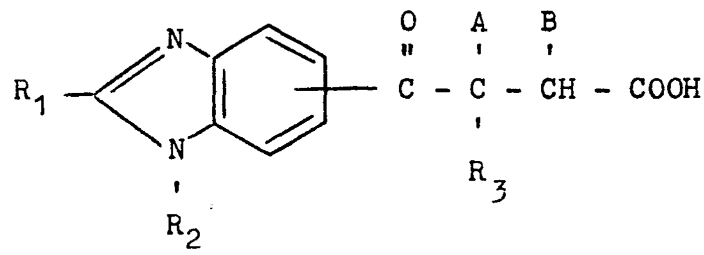

(III)

wherein

A, B and $R_1$ to $R_3$ are defined as in Claims 1 to 7, or an ester, amide or halide thereof, is reacted with hydrazine and, if desired, a compound of general formula I obtained according to process a or b wherein A and B each represents a hydrogen atom, is subsequently converted by dehydrogenation into a compound of general formula I, wherein A and B together represent another bond, and/or a compound of general formula I obtained, wherein $R_1$ represents the hydroxy or mercapto group or a phenyl group, which is mono-, di- or trisubstituted by a hydroxy and/or mercapto group, and/or $R_2$ represents a hydrogen atom, is converted by alkylation into a corresponding alkoxy, alkylmercapto and/or alkyl compound of general formula I, and/or a compound of general formula I obtained, wherein $R_1$ represents a phenyl group substituted by at least one alkylmercapto group, is converted by oxidation into a corresponding alkylsulfinylphenyl compound of general formula I, and/or a compound of general formula I obtained, wherein $R_1$ represents an alkylmercapto group, is converted into a corresponding hydroxy compound of general formula I, by oxidation and subsequent hydrolytic removal of the alkylsulfinyl or alkylsulfonyl group formed, and/or a racemic mixture of a compound of general formula I obtained, where A and B each represents a hydrogen atom, is resolved into the optically active antipodes thereof, and/or a compound of general formula I obtained is converted into the physiologically acceptable acid addition salts thereof with inorganic or organic acids.

**Claims** (for the Contracting state : AT)

1. Process for the preparation of new benzimidazoles of general formula

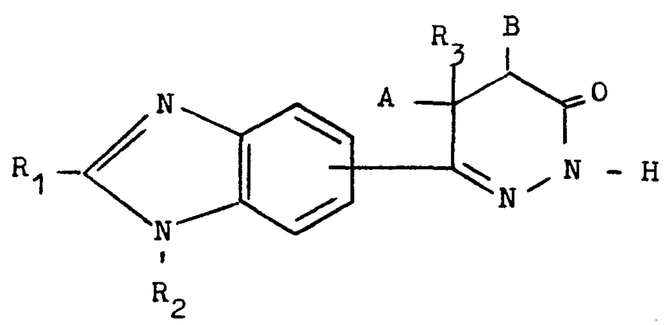

(I)

substituted in the 5 or 6 position by a pyridazinone ring,

wherein

A and B each represents a hydrogen atom or together represent another bond,

$R_1$ represents a hydrogen atom, the trifluoromethyl group, an alkyl group with 1 to 11 carbon atoms, a cycloalkyl group with 3 tô 7 carbon atoms, a hydroxy or mercapto group optionally substituted by an alkyl group with 1 to 6 carbon atoms, a phenylalkyl group with 1 to 3 carbon atoms in the alkyl part or a phenyl group, which may be mono-, di or trisubstituted by a halogen atom, an alkyl group with 1 to 4 carbon atoms, an alkylsulfinyl group with 1 to 6 carbon atoms and/or a hydroxy or mercapto group optionally substituted by an alkyl group with 1 to 6 carbon atoms, wherein the substituents of the phenyl nucleus may be the same or different,

$R_2$ represents a hydrogen atom, an alkyl group with 1 to 5 carbon atoms, a cycloalkyl group with 3 to 6 carbon atoms or a phenylalkyl group with 1 to 3 carbon atoms in the alkyl part and

$R_3$ represents an alkyl group with 1 to 6 carbon atoms, and the optically active antipodes thereof, where A and B each represents a hydrogen atom, and the physiologically acceptable acid addition salts thereof with inorganic or organic acids, characterised in that

a) a çompound of general formula II, optionally prepared in the reaction mixture,

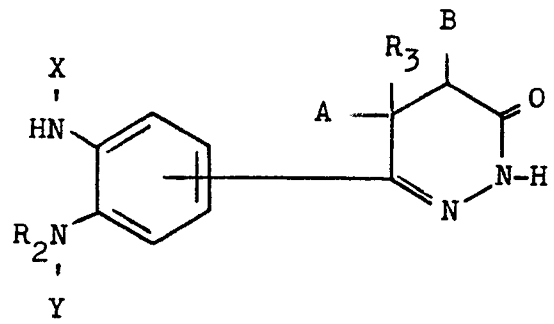

(II)

wherein

A, B, $R_2$ and $R_3$ are as hereinbefore defined, one of the radicals X or Y represents a hydrogen atom and the other of the radicals X or Y or both radicals X and Y each represents a group of formula

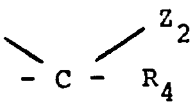

wherein

$Z_1$ and $Z_2$, which may be the same or different, represent optionally substituted amino groups or hydroxy or mercapto groups optionally substituted by a lower alkyl group, or

$Z_1$ and $Z_2$ together represent an oxygen or sulfur atom, an imino group optionally substituted by an alkyl group with 1 to 3 carbon atoms, an alkylenedioxy or alkylenedithio group, each with 2 or 3 carbon atoms and

$R_4$ has the meanings given above for $R_1$, or represents an amino group optionally substituted by an alkyl group with 1 to 3 carbon atoms, or the alkali salts thereof, if $R_4$ represents the mercapto group, is cyclised, or

b) a carboxylic acid of general formula

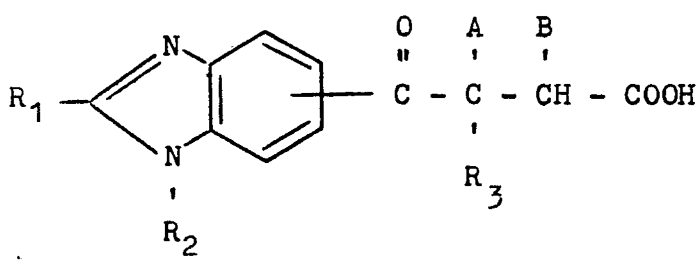

(III)

wherein

A, B and $R_1$ to $R_3$ are as hereinbefore defined, or an ester, amide or halide thereof, is reacted with hydrazine and, if desired, a compound of general formula I, obtained according to process a) or b), wherein A and B each represents a hydrogen atom, is subsequently converted, by dehydrogenation, into a compound of general formula I, wherein A and B together represent another bond, and/or a compound of general formula I obtained, wherein $R_1$ represents the hydroxy or mercapto group or a phenyl group, which is mono-, di- or trisubstituted by a hydroxy and/or mercapto group, and/or $R_2$ represents a hydrogen atom, is converted by alkylation into a corresponding alkoxy, alkylmercapto and/or alkyl compound of general formula I, and/or a compound of general formula I obtained wherein $R_1$ represents a phenyl group substituted by at least one alkylmercapto group is converted by oxidation into a corresponding alkylsulfinylphenyl compound of general formula I, and/or a compound of general formula I obtained, wherein $R_1$ represents an alkylmercapto group, is converted into a corresponding hydroxy compound of general formula I by oxidation and subsequent hydrolytic removal of the alkylsulfinyl or alkylsulfonyl group formed, and/or a racemic mixture of a compound of general formula I obtained, where A and B each represents a hydrogen atom, is resolved into the optically active antipodes thereof, and/or a compound of general formula I obtained is converted into the physiologically acceptable acid addition salts thereof with inorganic or organic acids.

2. Process as claimed in claim 1 for the preparation of new benzimidazoles of general formula

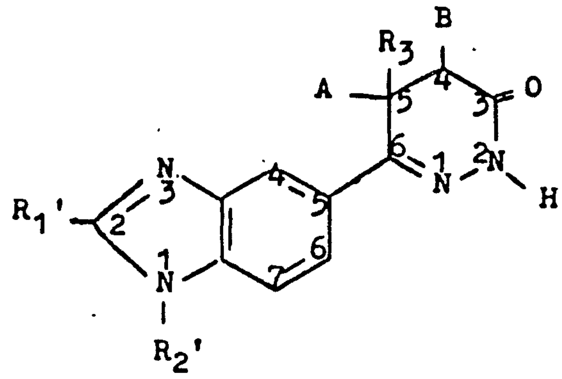

(Ia)

substituted in the 5 position by a pyridazinone ring,
wherein

$R_3$, A and B are defined as in claim 1,

$R_1'$ represents a hydrogen atom, the trifluoromethyl group, an alkyl group with 1 to 6 carbon atoms, a cycloalkyl group with 3 to 7 carbon atoms, a hydroxy or mercapto group optionally substituted by an alkyl group with 1 to 6 carbon atoms, or a phenyl group which may be mono-, di- or trisubstituted by a halogen atom, an alkyl group with 1 to 4 carbon atoms and/or a hydroxy or mercapto group optionally substituted by an alkyl group with 1 to 3 carbon atoms, wherein the substituents of the phenyl nucleus may be the same or different, and

$R_2'$ represents a hydrogen atom, an alkyl group with 1 to 3 carbon atoms or a cycloalkyl group with 3 to 6 carbon atoms, and of the optically active antipodes thereof, where A and B each represents a hydrogen atom, the 1H and 3H tautomers thereof and the physiologically acceptable acid addition salts thereof with inorganic or organic acids, characterised in that

a) a compound of general formula IIa,

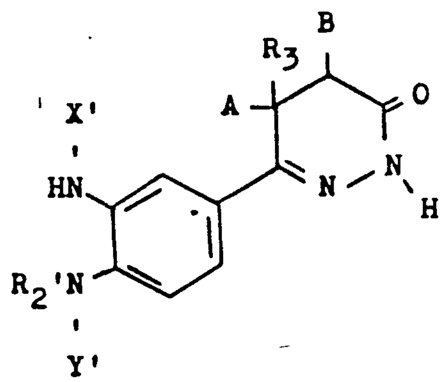

(IIa)

optionally prepared in the reaction mixture,
wherein

A, B, $R_2'$ and $R_3$ are as hereinbefore defined, one of the groups X' or Y' represents a hydrogen atom and the other group X' or Y' represents a group of formula

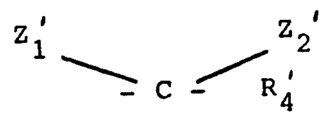

wherein

$Z_1'$ and $Z_2'$, which may be the same or different, represent hydroxy or mercapto groups optionally substituted by lower alkyl groups, or

$Z_1'$ and $Z_2'$ together represent an oxygen or sulphur atom, an imino group optionally substituted by an alkyl group with 1 to 3 carbon atoms, an alkylenedioxy or alkylenedithio group, each with 2 or 3 carbon atoms, and

$R_4'$ has the meanings given above for $R_1'$ or represents an amino group optionally substituted by an alkyl group with 1 to 3 carbon atoms, or the alkali salts thereof, where $R_4'$ represents the mercapto group, is cyclised, or

b) a carboxylic acid of general formula

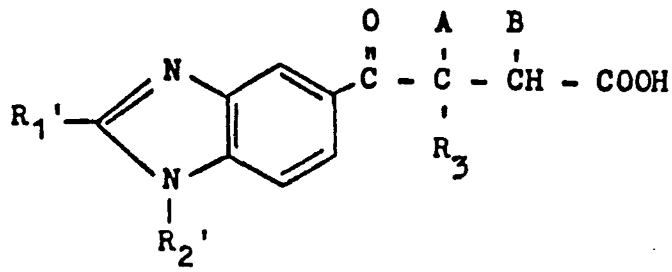

(IIIa)

wherein

A, B, $R_1'$, $R_2'$ and $R_3$ are as hereinbefore defined, or an ester, amide or halide thereof, is reacted with hydrazine, and subsequently if desired, a compound of general formula (Ia) obtained according to process a) or b), wherein A and B each represents a hydrogen atom, is converted by dehydrogenation into a compound of general formula (Ia) wherein A and B together represent another bond, and/or a compound of general formula (Ia) obtained, wherein $R_1'$ represents the hydroxy or mercapto group or a phenyl group which is mono-, di- or trisubstituted by a hydroxy and/or mercapto group, and/or $R_2'$ represents a hydrogen atom, is converted by alkylation into a corresponding alkoxy, alkylmercapto and/or alkyl compound of general formula (Ia), and/or a compound of general formula (Ia) obtained, wherein $R_1'$ represents an alkylmercapto group, is converted into a corresponding hydroxy compound of general formula (Ia) by oxidation and subsequent hydrolytic removal of the alkylsulfinyl or alkylsulfonyl group formed, and/or a racemic mixture of a compound of general formula (Ia) obtained is resolved into the optically active antipodes thereof, and/or a compound of general formula (Ia) obtained is converted into the physiologically acceptable acid addition salts thereof with inorganic or organic acids.

3. Process as claimed in claim 1 for the preparation of new benzimidazoles of general formula

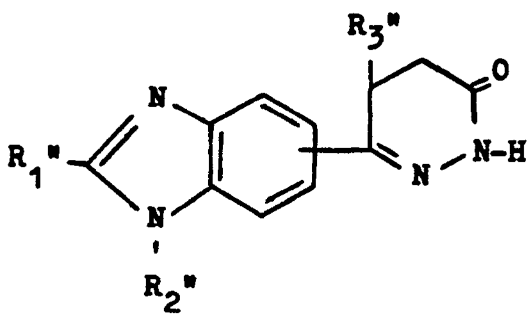

(Ib)

substituted in the 5 or 6 position by a pyridazinone ring,

wherein

$R_1''$ represents a methyl, trifluoromethyl, cycloheptyl, ethoxy, isopropylmercapto, methoxyphenyl, hydroxyphenyl, trimethoxyphenyl or bromo-methoxyphenyl group, a phenylalkyl group with 1 to 3 carbon atoms in the alkyl part, an alkyl group with 7 to 11 carbon atoms, an alkoxyphenyl group with 4 to 6 carbon atoms in the alkoxy part, or a phenyl or methoxyphenyl group mono- or disubstituted by alkylsulfinyl groups with 1 to 6 carbon atoms in the alkyl part,

$R_2''$ represents a hydrogen atom, a methyl or cyclohexyl group, an alkyl group with 4 or 5 carbon atoms or a phenylalkyl group with 1 to 3 carbon atoms in the alkyl part, and

$R_3''$ represents a methyl, ethyl or butyl group, and of the physiologically acceptable acid addition salts thereof with inorganic or organic acids, characterised in that

a) a compound of general formula

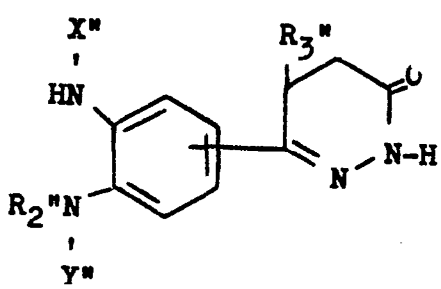

(IIb)

optionally prepared in the reaction mixture,

wherein

$R_2''$ and $R_3''$ are as hereinbefore defined, one of the groups X'' and Y'' represents a hydrogen atom and the other group X'' or Y'' represents a group of formula

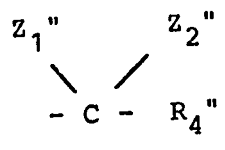

wherein

$Z_1''$ and $Z_2''$, which may be identical or different, represent optionally substituted amino groups or hydroxy or mercapto groups optionally substituted by lower alkyl groups, or

$Z_1''$ and $Z_2''$ together represent an oxygen or sulphur atom, an imino group optionally substituted by an alkyl group with 1 to 3 carbon atoms, an alkylenedioxy or alkylenedithio group, each with 2 or 3 carbon atoms, and

$R_4''$ has the meanings given for $R_1''$ hereinbefore, or represents an amino group optionally substituted by an alkyl group with 1 to 3 carbon atoms, or the alkali salts thereof, if $R_4''$ represents the mercapto group, is cyclised, or

b) a carboxylic acid of general formula

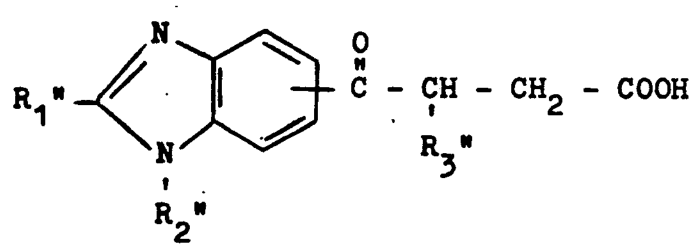

(IIIb)

wherein

$R_1''$ to $R_3''$ are as hereinbefore defined, or an ester, amide or halide thereof, is reacted with hydrazine, and subsequently, if desired, a compound of general formula (Ib) obtained according to process a) or b), wherein $R_1''$ represents the hydroxy or mercapto group or a phenyl group which is mono-, di- or trisubstituted by a hydroxy and/or mercapto group, and/or $R_2''$ represents a hydrogen atom, is converted by alkylation into a corresponding alkoxy, alkylmercapto and/or alkyl compound of general formula (Ib), and/or a compound of general formula (Ib) obtained, wherein $R_1''$ represents a phenyl group substituted by at least one alkylmercapto group, is converted by oxidation into a corresponding alkylsulfinyl compound of general formula (Ib) and/or a racemic mixture of a compound of general formula (Ib) obtained is resolved into the optically active antipodes thereof, and/or a compound of general formula (Ib) obtained is converted into the physiologically acceptable acid addition salts thereof with inorganic or organic acids.

Priority : 01.06.1979 (P 29 22 336.7)

4. Process as claimed in claims 1, 2 and 3, characterised in that the reaction is effected in a solvent and at temperatures of between 0 and 250 °C.

5. Process as claimed in claims 1a, 2a, 3a and 4 characterised in that the cyclisation is effected in the presence of a condensing agent or in the presence of a base.

6. Process as claimed in claims 1b, 2b and 3b, characterised in that the reaction is effected in a solvent at temperatures of between 0 and 150 °C.

7. Process as claimed in claims 1b, 2b, 3b and 6, characterised in that the reaction is effected in the presence of an acidic condensing agent.

**Revendications** (pour les Etats contractants : BE, CH, DE, FR, GB, IT, LU, NL, SE)

1. Nouveaux benzimidazoles substitués en position 5 ou 6 par un cycle pyridazinone, de formule générale :

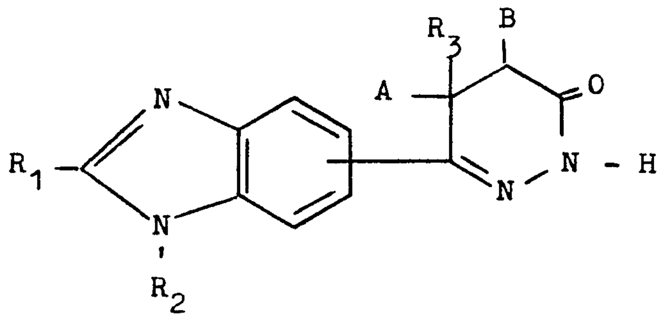

(I)

dans laquelle

A et B représentent chacun un atome d'hydrogène ou ensemble une autre liaison,

$R_1$ représente un atome d'hydrogène, le groupe trifluorométhyle, un groupe alcoyle avec 1 à 11 atomes de carbone, un groupe cycloalcoyle avec 3 à 7 atomes de carbone, un groupe hydroxy ou mercapto éventuellement substitué par un groupe alcoyle avec 1 à 6 atomes de carbone, un groupe phénylalcoyle avec 1 à 3 atomes de carbone dans la partie alcoyle ou un groupe phényle qui peut être mono-, di- ou trisubstitué par un atome d'halogène, un groupe alcoyle avec 1 à 4 atomes de carbone, un groupe alcoylsulfinyle avec 1 à 6 atomes de carbone et/ou un groupe hydroxy ou mercapto éventuellement substitué par un groupe alcoyle avec 1 à 6 atomes de carbone, les substituants du noyau phényle pouvant être identiques ou différents,

$R_2$ représente un atome d'hydrogène, un groupe alcoyle avec 1 à 5 atomes de carbone, un groupe cycloalcoyle avec 3 à 6 atomes de carbone ou un groupe phénylalcoyle avec 1 à 3 atomes de carbone dans la partie alcoyle et

$R_3$ représente un groupe alcoyle avec 1 à 6 atomes de carbone, leurs antipodes optiquement actifs, lorsque A et B représentent chacun un atome d'hydrogène, et leurs sels physiologiquement supportables d'addition avec des acides minéraux ou organiques.

2. Nouveaux benzimidazoles de formule générale I selon la revendication 1, dans laquelle

A et B représentent chacun un atome d'hydrogène ou ensemble une autre liaison,

$R_1$ représente un atome d'hydrogène, le groupe trifluorométhyle, un groupe alcoyle avec 1 à 11 atomes de carbone, un groupe cycloalcoyle avec 3 à 7 atomes de carbone, un groupe hydroxy éventuellement substitué par un groupe alcoyle avec 1 à 3 atomes de carbone, un groupe mercapto éventuellement substitué par un groupe alcoyle avec 1 à 6 atomes de carbone, un groupe phénylalcoyle avec 1 à 3 atomes de carbone dans la partie alcoyle, un groupe phényle éventuellement substitué par un groupe hydroxy, un atome d'halogène, un groupe alcoyle avec 1 à 4 atomes de carbone ou un groupe alcoxy avec 1 à 6 atomes de carbone, un groupe phényle substitué par un groupe méthoxy et par un atome d'halogène, un groupe méthylmercapto ou méthylsulfinyle ou un groupe phényle substitué par 2 ou 3 groupes méthoxy,

$R_2$ représente un atome d'hydrogène, un groupe alcoyle avec 1 à 5 atomes de carbone, un groupe cyclopropyle, cyclohexyle ou benzyle et

$R_3$ représente un groupe alcoyle avec 1 à 4 atomes de carbone, leurs antipodes optiquement actifs, lorsque A et B représentent chacun un atome d'hydrogène, et leurs sels physiologiquement supportables d'addition avec des acides minéraux ou organiques.

3. Nouveaux benzimidazoles de formule générale I selon la revendication 1, dans laquelle

A et B représentent chacun un atome d'hydrogène ou ensemble une autre liaison,

$R_1$ représente un atome d'hydrogène, le groupe méthyle, isopropyle, n-pentyle, 2-pentyle, n-hexyle, n-undécyle, cyclopropyle, cyclohexyle, cycloheptyle, hydroxy, éthoxy, mercapto, méthylmercapto, isopropylmercapto, n-hexylmercapto, trifluorométhyle, 1-phényléthyle, 1-phényl-2-propyle, phényle, 2-fluorophényle, 4-hydroxyphényle, 2-méthoxyphényle, 3-méthoxyphényle, 4-méthoxyphényle, 2,4-diméthoxyphényle, 3,4-diméthoxyphényle, 3,4,5-triméthoxyphényle, 4-n-hexyloxyphényle, 4-méthylphényle, 4-tert.butylphényle, 4-chlorophényle, 2-méthoxy-4-méthylmercaptophényle, 2-méthoxy-4-méthylsulfinylphényle ou 2-bromo-5-méthoxyphényle,

$R_2$ représente un atome d'hydrogène, le groupe méthyle, 3-méthylbutyle, cyclopropyle, cyclohexyle ou benzyle et

$R_3$ représente le groupe méthyle, éthyle ou n-butyle, leurs antipodes optiquement actifs lorsque A et B représentent chacun un atome d'hydrogène, et leurs sels physiologiquement supportables d'addition avec des acides minéraux ou organiques.

4. Nouveaux benzimidazoles de formule générale I selon la revendication 1, dans laquelle

A et B représentent chacun un atome d'hydrogène ou ensemble une autre liaison,

$R_1$ représente un atome d'hydrogène, le groupe méthyle, 2-pentyle, 1-phényléthyle, éthoxy, méthylmercapto, 4-méthylphényle, 2-fluorophényle, 4-méthoxyphényle, 4-hexyloxyphényle ou 2,4-diméthoxyphényle,

$R_2$ représente un atome d'hydrogène, le groupe méthyle ou benzyle et

$R_3$ représente le groupe méthyle, leurs antipodes optiquement actifs lorsque A et B représentent chacun un atome d'hydrogène, et leurs sels physiologiquement supportables d'addition avec des acides minéraux ou organiques.

5. Nouveaux benzimidazoles de formule générale I selon la revendication 1, dans laquelle

$R_1$, $R_2$ et $R_3$ sont définis comme à la revendication 2 et

A et B représentent chacun un atome d'hydrogène, leurs antipodes optiquement actifs et leurs sels physiologiquement supportables d'addition avec des acides minéraux ou organiques.

6. Le 2-(4-méthoxy-phényl)-5(6)-(5-méthyl-3-oxo-4,5-dihydro-2H-6-pyridazinyl)-benzimidazole, ses antipodes optiquement actifs et sels d'addition avec des acides.

7. Le 1-benzyl-2-méthyl-5-(5-méthyl-3-oxo-4,5-dihydro-2H-6-pyridazinyl)-benzimidazole, ses antipodes optiquement actifs et sels d'addition avec des acides.

8. Benzimidazoles selon les revendications 1 à 7 pour l'utilisation dans la lutte contre les maladies cardiovasculaires, les ulcères, les virus et les maladies virales.

9. Médicament contenant un composé selon les revendications 1 à 7 conjointement à un ou plusieurs excipients et/ou diluants inertes.

10. Procédé pour la préparation des nouveaux benzimidazoles substitués en position 5 ou 6 par un cycle pyridazinone de formule générale I des revendications 1 à 7, caractérisé en ce que

a) on cyclise un composé éventuellement préparé dans le mélange réactionnel, de formule générale

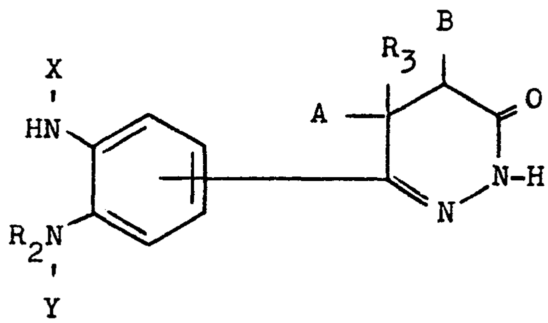

(II)

dans laquelle

A, B, $R_2$ et $R_3$ sont définis comme dans les revendications 1 à 7, l'un des radicaux X ou Y représente un atome d'hydrogène et l'autre radical X ou Y ou les deux radicaux X et Y représentent chacun un groupe de formule

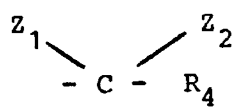

dans laquelle

$Z_1$ et $Z_2$ qui peuvent être identiques ou différents, représentent des groupes amino éventuellement substitués ou des groupes hydroxy ou mercapto éventuellement substitués par des groupes alcoyle inférieur ou

$Z_1$ et $Z_2$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone et

$R_4$ possède les significations données au début pour $R_1$ dans les revendications 1 à 7 ou représente un groupe amino éventuellement substitué par un groupe alcoyle avec 1 à 3 atomes de carbone, ou ses sels alcalins, lorsque $R_4$ représente le groupe mercapto ou

b) on fait réagir un acide carboxylique de formule générale

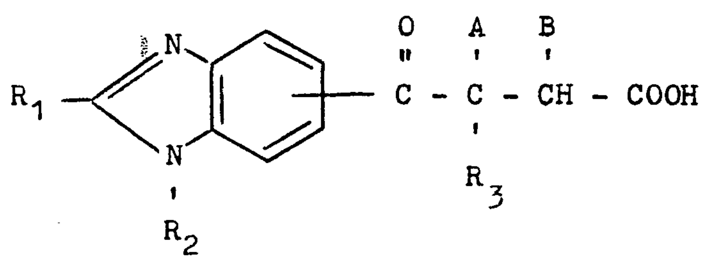

(III)

dans laquelle

A, B et $R_1$ à $R_3$ sont définis comme dans les revendications 1 à 7, ou leurs esters, amides ou halogénures avec l'hydrazine et éventuellement on transforme ensuite, par déshydrogénation, un composé de formule générale I obtenu selon les procédés a ou b, dans lequel A et B représentent chacun un atome d'hydrogène, en un composé de formule générale I dans laquelle A et B représentent ensemble une autre liaison et/ou on transforme, par alcoylation, un composé obtenu de formule générale I, dans laquelle $R_1$ représente le groupe hydroxy ou mercapto ou un groupe phényle qui est mono-, di- ou trisubstitué par un groupe hydroxy et/ou mercapto, et/ou $R_2$ représente un atome d'hydrogène, en un composé alcoxy, alcoylmercapto et/ou alcoylé correspondant de formule générale I et/ou on transforme, par oxydation, un composé obtenu de formule générale I dans laquelle $R_1$ représente un groupe phényle substitué par au moins un groupe alcoylmercapto, en un composé alcoylsulfinylphénylé correspondant de formule générale I et/ou on transforme un composé obtenu de formule générale I dans laquelle $R_1$ représente un groupe alcoylmercapto, par oxydation et clivage hydrolytique subséquent du groupe alcoylsulfinyle ou respectivement alcoylsulfonyle formé, en un composé hydroxy correspondant de formule générale I et/ou on sépare en ses antipodes optiquement actifs un mélange racémique obtenu d'un composé de formule générale I, lorsque A et B représentent chacun un atome d'hydrogène, par clivage de racémate, et/ou on transforme un composé obtenu de formule générale I en ses sels physiologiquement supportables d'addition avec des acides minéraux ou organiques.

**Revendications** (pour l'Etat contractant : AT)

1. Procédé pour la préparation de nouveaux benzimidazoles substitués en position 5 ou 6 par un cycle pyridazinone de formule générale

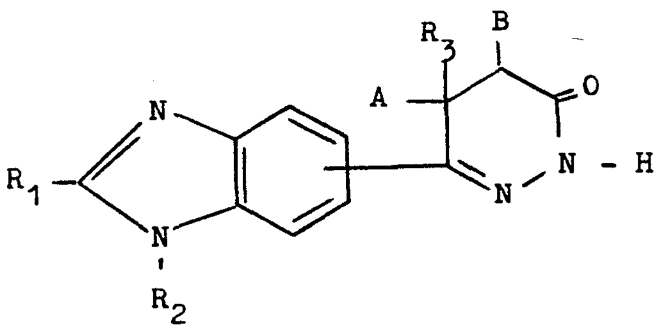

(I)

dans laquelle

A et B représentent chacun un atome d'hydrogène ou ensemble une autre liaison,

$R_1$ représente un atome d'hydrogène, le groupe trifluorométhyle, un groupe alcoyle avec 1 à 11 atomes de carbone, un groupe cycloalcoyle avec 3 à 7 atomes de carbone, un groupe hydroxy ou mercapto éventuellement substitué par un groupe alcoyle avec 1 à 6 atomes de carbone, un groupe phénylalcoyle avec 1 à 3 atomes de carbone dans la partie alcoyle ou un groupe phényle qui peut être mono-, di- ou trisubstitué par un atome d'halogène, un groupe alcoyle avec 1 à 4 atomes de carbone, un groupe alcoylsulfinyle avec 1 à 6 atomes de carbone et/ou un groupe hydroxy ou mercapto éventuellement substitué par un groupe alcoyle avec 1 à 6 atomes de carbone, les substituants du noyau phényle pouvant être identiques ou différents,

$R_2$ représente un atome d'hydrogène, un groupe alcoyle avec 1 à 5 atomes de carbone, un groupe cycloalcoyle avec 3 à 6 atomes de carbone ou un groupe phénylalcoyle avec 1 à 3 atomes de carbone dans la partie alcoyle et

$R_3$ représente un groupe alcoyle avec 1 à 6 atomes de carbone, ainsi que de leurs antipodes optiquement actifs, lorsque A et B représentent chacun un atome d'hydrogène, et de leurs sels physiologiquement supportables d'addition avec des acides minéraux ou organiques, caractérisé en ce que

a) on cyclise un composé éventuellement préparé dans le milieu réactionnel de formule générale

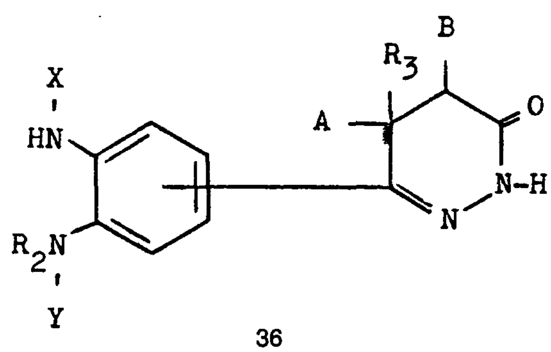

(II)

dans laquelle

A, B, $R_2$ et $R_3$ sont définis comme précédemment, l'un des radicaux X ou Y représente un atome d'hydrogène et l'autre radical X ou Y ou les deux radicaux X et Y représentent chacun un groupe de formule

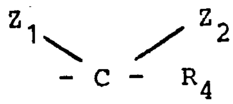

dans laquelle

$Z_1$ et $Z_2$ qui peuvent être identiques ou différents, représentent des groupes amino éventuellement substitués ou des groupes hydroxy ou mercapto éventuellement substitués par des groupes alcoyle inférieur ou

$Z_1$ et $Z_2$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone et

$R_4$ possède les significations données au début pour $R_1$ ou représente un groupe amino éventuellement substitué par un groupe alcoyle avec 1 à 3 atomes de carbone, ou ses sels alcalins, lorsque $R_4$ représente le groupe mercapto ou

b) on fait réagir un acide carboxylique de formule générale

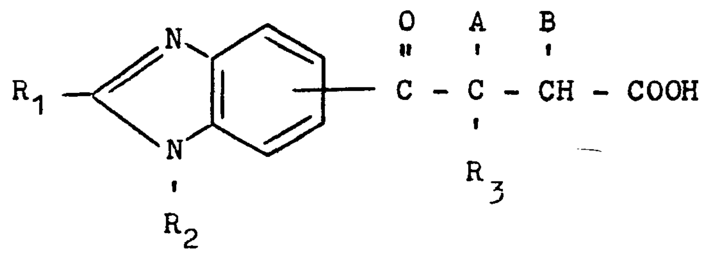

(III)

dans laquelle

A, B et $R_1$ à $R_3$ sont définis comme au début, ou ses esters, amides ou halogénures avec l'hydrazine, et éventuellement on transforme ensuite, par déshydrogénation, un composé de formule générale (I) obtenu selon les procédés a) ou b), dans lequel A et B représentent chacun un atome d'hydrogène, en un composé de formule générale (I) dans laquelle A et B représentent ensemble une autre liaison et/ou on transforme, par alcoylation, un composé obtenu de formule générale (I), dans laquelle $R_1$ représente le groupe hydroxy ou mercapto ou un groupe phényle qui est mono-, di- ou trisubstitué par un groupe hydroxy et/ou mercapto, et/ou $R_2$ représente un atome d'hydrogène, en un composé alcoxy, alcoylmercapto et/ou alcoylé correspondant de formule générale (I) et/ou on transforme, par oxydation, un composé obtenu de formule générale (I) dans laquelle $R_1$ représente un groupe phényle substitué par au moins un groupe alcoylmercapto en un composé alcoylsulfinylphénylé correspondant de formule générale (I) et/ou on transforme un composé obtenu de formule générale (I) dans laquelle $R_1$ représente un groupe alcoylmercapto, par oxydation et clivage hydrolytique subséquent du groupe alcoylsulfinyle ou respectivement alcoylsulfonyle formé, en un composé hydroxy correspondant de formule générale (I) et/ou on sépare en ses antipodes optiquement actifs un mélange racémique obtenu d'un composé de formule générale (I), lorsque A et B représentent chacun un atome d'hydrogène, par clivage de racémate et/ou on transforme un composé obtenu de formule générale (I) en ses sels physiologiquement supportables d'addition avec des acides minéraux ou organiques.

2. Procédé selon la revendication 1 pour la préparation de nouveaux benzimidazoles substitués en position 5 par un cycle pyridazinone de formule générale

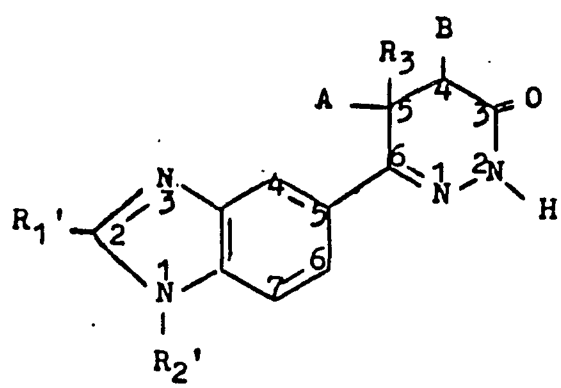

(Ia)

dans laquelle

$R_3$, A et B sont définis comme dans la revendication 1,

$R_1'$ représente un atome d'hydrogène, le groupe trifluorométhyle, un groupe alcoyle avec 1 à 6 atomes de carbone, un groupe cycloalcoyle avec 3 à 7 atomes de carbone, un groupe hydroxy ou mercapto éventuellement substitué par un groupe alcoyle avec 1 à 6 atomes de carbone ou un groupe phényle qui peut être mono-, di- ou trisubstitué par un atome d'halogène, un groupe alcoyle avec 1 à 4 atomes de carbone et/ou un groupe hydroxy ou mercapto éventuellement substitué par un groupe alcoyle avec 1 à 3 atomes de carbone, les substituants du noyau phényle pouvant être identiques ou différents, et

$R_2'$ représente un atome d'hydrogène, un groupe alcoyle avec 1 à 3 atomes de carbone ou un groupe cycloalcoyle avec 3 à 6 atomes de carbone, ainsi que de leurs antipodes optiquement actifs, lorsque A et B représentent chacun un atome d'hydrogène, de leurs tautomères 1H et 3H et de leurs sels physiologiquement supportables d'addition avec des acides minéraux ou organiques, caractérisé en ce que

a) on cyclise un composé éventuellement préparé dans le mélange réactionnel de formule générale

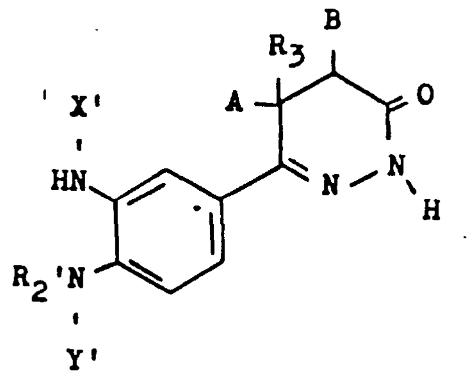

(IIa)

dans laquelle

A, B, $R_2'$ et $R_3$ sont définis comme au début, l'un des radicaux X' ou Y' représente un atome d'hydrogène et l'autre des radicaux X' ou Y' représente un groupe de formule

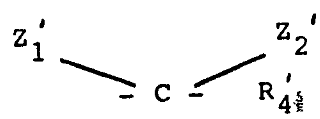

dans laquelle

$Z_1'$ et $Z_2'$, qui peuvent être identiques ou différents, représentent des groupes hydroxy ou mercapto éventuellement substitués par des groupes alcoyle inférieur ou

$Z_1'$ et $Z_2'$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone et

$R_4'$ possède les significations mentionnées au début pour $R_1'$ ou représente un groupe amino éventuellement substitué par un groupe alcoyle avec 1 à 3 atomes de carbone, ou ses sels alcalins lorsque $R_4'$ représente le groupe mercapto ou

b) on fait réagir un acide carboxylique de formule générale

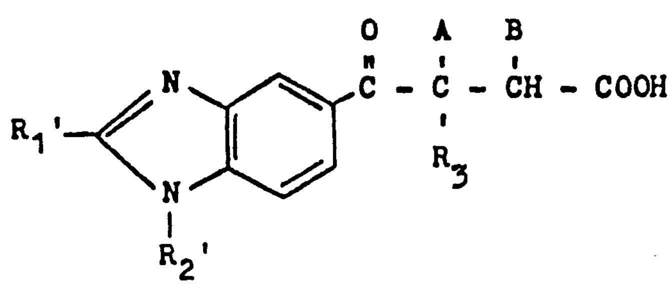

(IIIa)

dans laquelle

A, B, $R_1'$, $R_2'$ et $R_3$ sont définis comme au début, ou leurs esters, amides ou halogénures avec

l'hydrazine et éventuellement on transforme ensuite, par déshydrogénation, un composé obtenu selon les procédés a) ou b) de formule générale (Ia) dans laquelle A et B représentent chacun un atome d'hydrogène, en un composé de formule générale (Ia) dans laquelle A et B représentent ensemble une autre liaison, et/ou on transforme, par alcoylation, un composé obtenu de formule générale (Ia) dans laquelle $R_1'$ représente le groupe hydroxy ou mercapto ou un groupe phényle qui est mono-, di- ou trisubstitué par un groupe hydroxy et/ou mercapto, et/ou $R_2'$ représente un atome d'hydrogène, en un composé alcoxy, alcoylmercapto et/ou alcoylé correspondant de formule générale (Ia) et/ou on transforme un composé obtenu de formule générale (Ia), dans laquelle $R_1'$ représente un groupe alcoylmercapto, par oxydation et clivage hydrolytique subséquent du groupe alcoylsulfinyle ou alcoylsulfonyle formé, en un composé hydroxy correspondant de formule générale (Ia) et/ou on sépare un mélange racémique obtenu d'un composé de formule générale (Ia), par séparation de racémate, en ses antipodes optiquement actifs et/ou on transforme un composé obtenu de formule générale (Ia) en ses sels physiologiquement supportables d'addition avec des acides minéraux ou organiques.

3. Procédé selon la revendication 1 pour la préparation de nouveaux benzimidazoles substitués en position 5 ou 6 par un cycle pyridazinone de formule générale

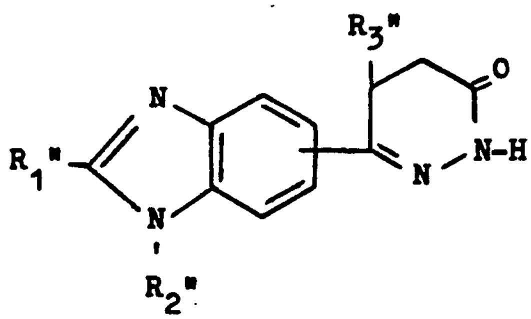

(Ib)

dans laquelle

$R_1''$ représente un groupe méthyle, trifluorométhyle, cycloheptyle, éthoxy, isopropylmercapto, méthoxyphényle, hydroxyphényle, triméthoxyphényle ou bromo-méthoxyphényle, un groupe phénylalcoyle avec 1 à 3 atomes de carbone dans la partie alcoyle, un groupe alcoyle avec 7 à 11 atomes de carbone, un groupe alcoxyphényle avec 4 à 6 atomes de carbone dans la partie alcoxy ou un groupe phényle ou méthoxyphényle mono- ou disubstitué par des groupes alcoylsulfinyle avec 1 à 6 atomes de carbone dans la partie alcoyle,

$R_2''$ représente un atome d'hydrogène, un groupe méthyle ou cyclohexyle, un groupe alcoyle avec 4 ou 5 atomes de carbone ou un groupe phénylalcoyle avec 1 à 3 atomes de carbone dans la partie alcoyle et

$R_3''$ représente un groupe méthyle, éthyle ou butyle, et de leurs sels physiologiquement supportables d'addition avec des acides minéraux ou organiques, caractérisé en ce que

a) on cyclise un produit éventuellement préparé dans le mélange réactionnel de formule générale

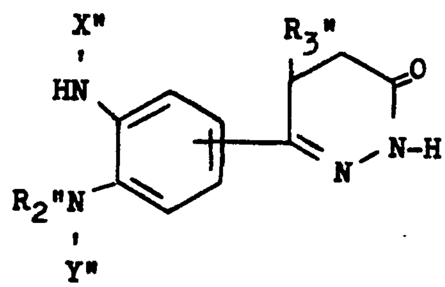

(IIb)

dans laquelle

$R_2''$ et $R_3''$ sont définis comme au début, l'un des radicaux X″ ou Y″ représente un atome d'hydrogène et l'autre des radicaux X″ ou Y″ représente un groupe de formule

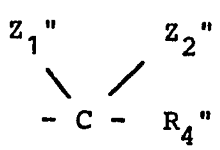

dans laquelle

$Z_1''$ et $Z_2''$ qui peuvent être identiques ou différents représentent des groupes amino éventuellement substitués ou des groupes hydroxy ou mercapto éventuellement substitués par des groupes alcoyle inférieur ou

$Z_1''$ et $Z_2''$ représentent ensemble un atome d'oxygène ou de soufre, un groupe imino éventuellement substitué par un groupe alcoyle avec 1 à 3 atomes de carbone, un groupe alcoylènedioxy ou alcoylènedithio avec dans chaque cas 2 ou 3 atomes de carbone et

$R_4''$ possède les significations données au début pour $R_1''$ ou représente un groupe amino éventuellement substitué par un groupe alcoyle avec 1 à 3 atomes de carbone, ou ses sels alcalins lorsque $R_4''$ représente le groupe mercapto ou on fait réagir un acide carboxylique de formule générale

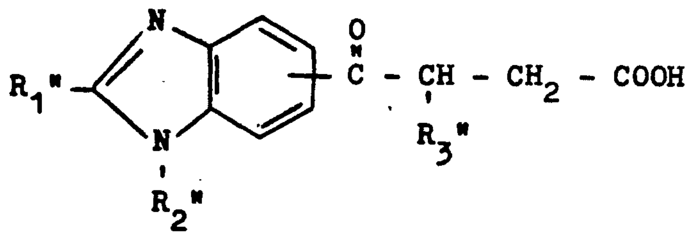

(IIIb)

dans laquelle

$R_1''$ à $R_3''$ sont définis comme au début, ou leurs esters, amides ou halogénures avec l'hydrazine et éventuellement on transforme ensuite, par alcoylation, un composé de formule générale (Ib) obtenu selon les procédés a) ou b) dans lequel $R_1''$ représente le groupe hydroxy ou mercapto ou un groupe phényle qui est mono-, di- ou trisubstitué par un groupe hydroxy et/ou mercapto, et/ou $R_2''$ représente un atome d'hydrogène, en un composé alcoxy, alcoylmercapto et/ou alcoylé correspondant de formule générale (Ib) et/ou on transforme, par oxydation, un composé obtenu de formule générale (Ib) dans laquelle $R_1''$ représente un groupe phényle substitué par au moins un groupe alcoylmercapto, en un composé alcoylsulfinylé correspondant de formule générale (Ib) et/ou sépare en ses antipodes optiquement actifs, par séparation de racémate, un mélange racémique obtenu d'un composé de formule générale (Ib) et/ou on transforme un composé obtenu de formule générale (Ib) en ses sels physiologiquement supportables d'addition avec des acides minéraux ou organiques.

Priorité : 01.06.1979 (P 29 22 336.7).

4. Procédé selon la revendication 1, 2 et 3, caractérisé en ce que la réaction est effectuée dans un solvant et à des températures comprises entre 0 et 250 °C.

5. Procédé selon la revendication 1a, 2a, 3a et 4, caractérisé en ce que la cyclisation est effectuée en présence d'un agent de condensation ou en présence d'une base.

6. Procédé selon la revendication 1b, 2b et 3b, caractérisé en ce que la réaction est effectuée dans un solvant à des températures comprises entre 0 et 150 °C.

7. Procédé selon la revendication 1b, 2b, 3b et 6, caractérisé en ce que la réaction est effectuée en présence d'un agent de condensation acide.